# EUROPEAN PATENT SPECIFICATION

(11) **EP 3 419 683 B1**
(45) Date of publication and mention of the grant of the patent: **06.10.2021**
(21) Application number: 17757208.8
(22) Date of filing: 23.02.2017
(51) Int. Cl.: A61K 31/095, A61K 9/08, A61K 47/02, A61K 47/12, A61K 47/18

(54) **NITRIC OXIDE GENERATING FORMULATIONS AND KITS**
STICKOXIDERZEUGENDE FORMULIERUNGEN UND KITS
FORMULATIONS GÉNÉRANT DE L'OXYDE NITRIQUE ET KITS

(30) Priority: 25.02.2016 US 201662299850 P
(43) Date of publication of application: 02.01.2019
(73) Proprietor: The Regents of The University of Michigan, Ann Arbor, Michigan 48109-2590 (US)
(72) Inventor: MEYERHOFF, Mark, E., Ann Arbor MI 48109-1055 (US); LAUTNER, Gergely, Ann Arbor MI 48109-1055 (US); BALIJEPALLI, Anant, Ann Arbor MI 48109 (US); SAJJAN, Umadeyi, Ann Arbor MI 48109 (US); REN, Hang, Ann Arbor MI 48109 (US)
(74) Representative: Forresters IP LLP
(86) International application number: PCT/US2017/019136
(87) International publication number: WO 2017/147295

(56) References cited:
- WO-A2-2008/153762
- WO-A2-2008/153762
- US-A1- 2003 215 528
- US-A1- 2004 037 897
- US-A1- 2009 170 932
- US-A1- 2009 287 072
- US-A1- 2009 287 072
- Holmes A.J.: "The role of L-ascorbic acid in S-nitrosothiol decomposition and aspects of thenitrosation of thiones, Durham theses, Durham University. Available at Durham E-Theses Online:http://etheses.dur.ac.uk/4228/", , 2000, XP002793842, Retrieved from the Internet: URL:http://etheses.dur.ac.uk/4228/ [retrieved on 2019-08-27]
- SMITH J.N. ET AL.: "Kinetics and mechanism of the decomposition of S-nitrosoglutathione by L-ascorbic acid and copper ions in aqueous solution to produce nitric oxide", NITRIC OXIDE: BIOLOGY AND CHEMISTRY, vol. 4, no. 1, 2000, pages 57-66, XP002793843,
- KASHIBA-IWATSUKI M. ET AL.: "Ascorbic acid and reducing agents regulate the fates and functions of S-nitrosothiols", J. BIOCHEM., vol. 122, 1997, pages 1208-1214, XP002793844,
- XU A. ET AL.: "Ascorbic acid and glutathione modulate the biological activity of S-nitrosoglutathione", HYPERTENSION, 2000, pages 291-295, XP002793845,

## Description

### BACKGROUND

In the human body, nitric oxide (NO) may be produced by any of several isoforms of the enzyme nitric oxide synthase (NOS). NO is central to the mammalian immune response or defense, and is a cytotoxic agent in the mechanisms used by macrophages to kill *L. major, M. bovis,* and *M*. *tuberculosis,* among numerous other species of bacteria. NO is also an effective antiviral agent, that has activity against the rhinovirus that causes common colds. NO is produced from L-arginine in the airways (e.g., in the upper respiratory tract) by immune cells (macrophages, neutrophils, lymphocytes, etc.) and airway epithelial cells (e.g., conductive and respiratory epithelial cells) primarily through inducible nitric oxide synthase (iNOS). Deficiencies in NO production can lead to decreased immune response and/or microbial biofilm formation. The deficiencies in nasal NO levels have been linked with diseases, such as primary ciliary dyskinesia and chronic rhinosinusitis (CRS), and potentially to the inability to fight viral agents that cause the common cold.

### SUMMARY

An example of a nitric oxide generating formulation includes an *S*-nitrosothiol (RSNO) powder, a salt, and an additive. The additive is to control or accelerate the rate of release of nitric oxide (NO) from RSNO after the RSNO powder, the salt, and additive are dissolved into a liquid carrier. The nitric oxide generating formulation may be part of a kit that includes a container for dissolving the components into a liquid carrier. The kit may be used to make an antimicrobial sinonasal treatment solution or a catheter lock solution.

### BRIEF DESCRIPTION OF THE DRAWINGS

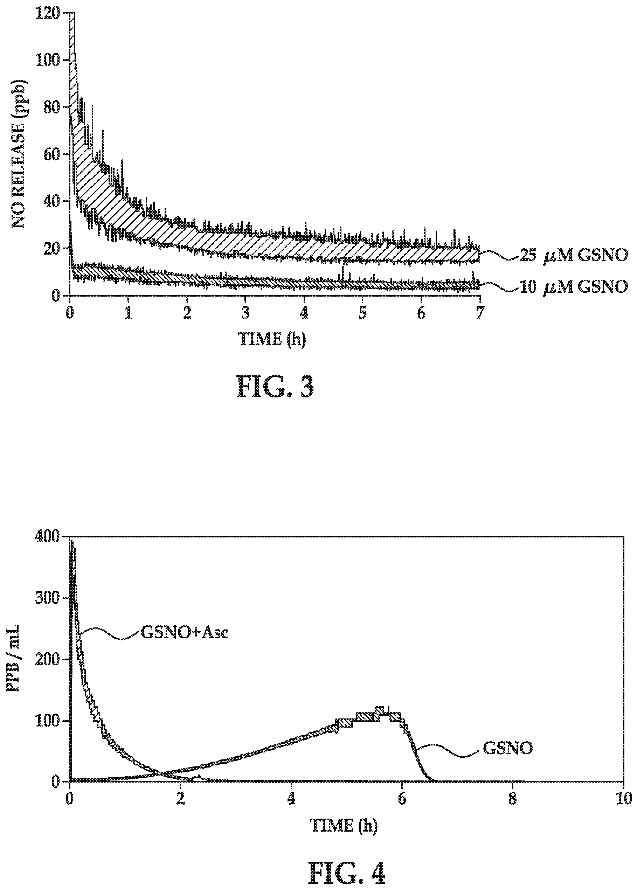

Features of examples of the present disclosure will become apparent by reference to the following detailed description and drawings. In some of the drawings (e.g., Figs. 3-5, 7, 9, 11, 13, 24, and 25), data is shown for nitric oxide (NO) release profiles or kinetics (e.g., ppb or PPB/mL; Y axis) as a function of time (X axis). In these drawings/figures, the data shows a wide band of values because of the high level of noise associated with chemiluminescence measurements. The noise may be due to aerosol droplets, from the nitrogen pursed solutions, which scatter light and increase the noise in the gas phase level that is detected by the optical photomultiplier detector within the chemiluminescence instrument.
Fig. 1 is a schematic illustration of an example of an antimicrobial sinonasal treatment solution (formed from an example of the antimicrobial sinonasal treatment formulation disclosed herein) being introduced into upper airways through a nasal cavity, where the enlarged illustration shows the production of nitric oxide gas (NO_{(g)}) from *S*-nitrosoglutathione (GSNO);
Fig. 2 is a schematic illustration of an example of a catheter lock solution being formed from an example of the catheter lock solution formulation disclosed herein and introduced into a catheter inserted into a blood vessel (e.g., artery or vein), where the enlarged illustration shows the production of nitric oxide gas (NO) from the NO donor in the lock solution;
Fig. 3 is a graph depicting the nitric oxide (NO) release profile from solutions including 10 µM GSNO and 25 µM GSNO in phosphate buffered saline (pH 7.4) over a 7 hour period;
Fig. 4 is a graph depicting the nitric oxide (NO) release kinetics of GSNO in saline (0.16 M NaCl + 0.03 M NaHCO₃ in Millipore purified water) both without Ascorbic acid (GSNO) and with Ascorbic acid (GSNO:Asc, 1:1 mole ratio; at 100 µM each);
Fig. 5 is a graph depicting the NO release kinetics of different concentrations of GSNO in saline (0.16 M NaCl + 0.065 M NaHCO₃ in Millipore water) at 37°C and at room temperature (e.g., from 20°C to 25°C);
Fig. 6 is a bar graph depicting the stability, in terms of % of initial GSNO versus time and as measured by UV/Vis spectrophotometry, of dry powder GSNO formulations when freshly prepared and after storage as dry powders at different temperatures for different time periods;
Fig. 7 is a graph depicting the NO release kinetics of GSNO:glutathione (GSH) (1:1 mole ratio; 100 µM each) in saline (0.16 M NaCl + 0.065 M NaHCO₃ in Millipore water) at 37°C, at room temperature (e.g., from 20°C to 25°C), and at 37°C in the presence of oxygen (air);
Fig. 8 is a bar graph depicting the stability, in terms of % of initial GSNO versus time and as measured by UV/Vis spectrophotometry, of GSNO:GSH (1:1 mole ratio) formulations when freshly prepared and after storage as dry powders at different temperatures for different time periods;
Fig. 9 is a graph depicting the NO release kinetics of GSNO:GSH: ethylenediaminetetraacetic acid (EDTA) (1:1:0.1 mole ratio; 100 µM of GSNO) in saline (0.16 M NaCl + 0.065 M NaHCO₃ in Millipore water) at 37°C and at room temperature (e.g., from 20°C to 25°C);
Fig. 10 is a bar graph depicting the stability, in terms of % of initial GSNO versus time and as measured by UV/Vis spectrophotometry, of GSNO:GSH:EDTA (1:1:0.1 mole ratio) formulations when freshly prepared and after storage as dry powers at different temperatures for different time periods;
Fig. 11 is a graph depicting the NO release kinetics of GSNO:Ascorbic acid (Asc) at different mole ratios in saline at 37°C and of GSNO:Asc (1:1 mole ratio; at 100 µM each) in saline at room temperature (e.g., from 20°C to 25°C);
Fig. 12 is a bar graph depicting the stability, in terms of % of initial GSNO versus time and as measured by UV/Vis, of GSNO:Asc (1:1 mole ratio) formulations when freshly prepared and after storage as dry powders at different temperatures for different time periods;
Fig. 13 is a graph depicting the NO release kinetics of freshly prepared GSNO:Asc:EDTA at different mole ratios in saline at 37°C and of GSNO:Asc:EDTA (1:1:0.1 mole ratio) in saline at room temperature (e.g., from 20°C to 25°C);
Fig. 14 is a bar graph depicting the stability, in terms of % of initial GSNO versus time and as measured by UV/Vis spectrophotometry, of GSNO:Asc:EDTA (1:1:0.1 mole ratio) formulations when freshly prepared and after storage as dry powders at different temperatures for different time periods;
Fig. 15 is a graph depicting the effect of various concentrations of GSNO:Asc (always at 1:1 mole ratio) on *S. aureus* biofilm disruption;
Fig. 16 is a graph depicting the effect of various concentrations of GSNO:Asc (always at 1:1 mole ratio) on *P. aeruginosa* biofilm disruption;
Fig. 17A is a graph depicting the effect of different concentrations of a GSNO:Asc (1:1 mole ratio) mixture on the killing *of S. aureus* biofilm grown on the apical surface of mucociliary-differentiated airway epithelial cell cultures established using cells obtained from cystic fibrosis (CF) patients (these cells are deficient in producing NO endogenously and therefore any nitrite detected is due to added GSNO:Asc);
Fig. 17B is a bar graph showing the levels of nitrite present on the apical surface of CF cell cultures colonized with *S. aureus* biofilm after treatment with different concentrations of GSNO:Asc;
Fig. 17C is a bar graph showing the release of interleukin-8 (IL-8), a pro-inflammatory cytokine, from CF airway epithelial cell cultures colonized with *S. aureus* and treated with different concentrations of GSNO:Asc;
Figs. 18A and 18B are confocal images (in black and white) illustrating that treatment with GSNO:Asc reduces the density of bacterial biofilm grown on the apical surface of mucociliary differentiated cell cultures when compared to mucociliary differentiated cell cultures treated with PBS;
Fig. 19 is a graph depicting the synergistic effect of GSNO:Asc (1:1 mole ratio with GSNO:Asc at 0.625 mM) and gentamicin (50 µg/ml) on the killing *of S. aureus* biofilm established on the apical surface of CF airway epithelial cell cultures;
Fig. 20 is a graph depicting the effect of GSH and ascorbic acid individually on the killing of colonized *S. aureus* on human CF epithelial cultures;
Fig. 21 is a graph depicting the effect of GSNO:Asc (1:1 mole ratio; at 0.625 mM GSNO:Asc) on the killing *of S. aureus* biofilm grown on the apical surface of sinonasal epithelial cell cultures established from patients with chronic rhinosinusitis;
Figs. 22A through 22D show scanning electron microscopy images *of S. aureus* biofilm grown on the apical surface of CF airway epithelial cell cultures prior to treatment with GSNO:Asc) (Figs. 20A and 20B) or 3 hours after treatment with GSNO:Asc (1:1 mole ratio; at 0.625 mM GSNO:Asc) (Figs. 3C and 3D);
Fig. 23 is a bar graph showing the ciliary beat frequency of uninfected CF cultures and CF cultures infected with *S. aureus* and then treated with either PBS or GSNO:Asc (1:1 mole ratio; at 0.625 mM GSNO:Asc);
Fig. 24 is a graph illustrating the outer surface NO flux, with respect to time, of a silicone rubber catheter filled with a GSNO:GSH lock solution (1:1 mole ratio, each at 2 mM concentration); and
Fig. 25 is a graph illustrating the outer surface NO flux, with respect to time, of a polyurethane catheter filled with a GSNO:GSH lock solution (1:1 mole ratio, each at 10 mM concentration).

### DETAILED DESCRIPTION

Nitric oxide (NO) is a potent antibacterial and antiviral agent. NO deficiencies *in vivo* can either be genetic or polymorphism-related, or induced by pathogens that take advantage of upstream regulation of NO production. Deficiencies in NO production can reduce mucociliary function (which is one of the primary innate immune defense mechanisms in the airway epithelium and is also directly correlated with ciliary beat frequency), increase susceptibility to microbial infections, and/or promote persistence of bacterial biofilm that are refractory to antibiotics.

In some of the examples disclosed herein, a nitric oxide generating formulation is specifically formulated as an antimicrobial sinonasal treatment formulation, which can be used to make an antimicrobial sinonasal treatment solution. The antimicrobial sinonasal treatment solution allows for the spontaneous delivery of NO slowly during a period in which residual sinonasal formulation resides in the sinus cavities. The antimicrobial sinonasal treatment solution disclosed herein increases NO levels outside and within epithelial cells and immune cells, which can also help control ciliary beat frequency. As such, the antimicrobial sinonasal treatment solution including an example of the nitric oxide generating formulation described herein may help to restore/enhance mucociliary function (which, as noted above, is directly correlated with ciliary beat frequency). Restored/enhanced mucociliary function may increase the defense against chronically colonized pathogens and reduce, or even prevent, disease perpetuation. In addition, the released nitric oxide from the treatment solution will have direct bactericidal and anti-viral activity on most types of bacteria and viruses that can infect the sinonasal cavities. Therefore, this sinonasal treatment solution may be beneficial for treating or even preventing upper airway infections, including CRS.

In other examples disclosed herein, the nitric oxide generating formulation is specifically formulated as a catheter lock solution formulation, which can be employed as a catheter lock solution. The catheter lock solution may be injected into lumen(s) of a catheter, where the solution spontaneously and slowly releases NO over a period of many hours to days. The NO can permeate through the walls of the catheter, and thus can help prevent adhesion and growth of a biofilm on both the inner lumen walls and outer surfaces of the catheter. As such, the catheter lock solution may be an antimicrobial catheter lock solution. In some of the examples disclosed herein, the catheter lock solution also includes additives that can serve as local anticoagulants or that can enhance the antithrombotic activity at the distal tip of the catheter.

Examples of the nitric oxide generating formulation disclosed herein include an *S-*nitrosothiol (RSNO) powder, a salt, and an additive. As described in further detail below, the components of the nitric oxide generating formulation may be part of a single composition (i.e., all mixed together in a single tablet, pellet, or powder mixture), or may be separate compositions that are to be mixed together in a liquid carrier. As examples of the separate compositions, the RSNO powder may be in one container or formulated as one pellet or tablet and the salt and additive may be together in another container or formulated as a separate pellet or tablet, or each of the RSNO powder, the salt, and the additive may be in separate containers or formulated as separate pellets or tablets.

The RSNO that is selected for the nitric oxide generating formulation is a species that is naturally occurring in the human body, or is capable of decomposing to a species that is naturally occurring in the human body, or is a drug that is suitable for human use (e.g., ingestion, consumption, etc.). In any of the examples disclosed herein, the RSNO or RSNO powder is selected from the group consisting of *S*-nitrosoglutathione (GSNO, naturally occurring in the human body), *S*-nitroso-cysteine (CYSNO, naturally occurring in the human body), *S*-nitroso-N-acetyl-penicillamine (SNAP, decomposes to the drug, penicillamine), *S*-nitroso-penicillamine, and S-nitroso-human serum albumin (naturally occurring in the human body).

*S*-nitrosoglutathione (GSNO) is one example of the NO releasing *S*-nitrosothiol (RSNO) molecule. GSNO exists in the human body as a result of NO (generated by endothelial cells, macrophages, sinus epithelial cells, etc.) reacting with oxygen to form N₂O₃, which can provide a nitrosonium ion (NO⁺) to react with the thiol group of glutathione to form GSNO. As such, the use of GSNO in the nitric oxide generating formulation disclosed herein does not introduce any foreign or toxic agents into the nasal cavity or into a blood vessel through the catheter.

GSNO may be prepared from glutathione (GSH) by acidifying a mixture of sodium nitrite/GSH with hydrochloric acid and then isolating the GSNO species (as solid crystals). Alternatively, the GSNO may be a commercially available sample.

In some examples, *S*-nitrosothiol (RSNO) molecules other than GSNO may be used in the nitric oxide generating formulation. Examples of these other *S*-nitrosothiols include *S-*nitroso-cysteine (CYSNO, naturally occurring in the human body), *S*-nitroso-N-acetyl-penicillamine (SNAP, decomposes to the drug, penicillamine), *S*-nitroso-penicillamine, and *S-*nitroso-human serum albumin (naturally occurring in the human body).

When the nitric oxide generating formulation is the antimicrobial sinonasal treatment formulation, any salt that provides physiological osmolarity may be used. Examples of suitable salts for the antimicrobial sinonasal treatment formulation are selected from the group consisting of sodium chloride, sodium bicarbonate, calcium chloride, a sodium phosphate buffer, a potassium phosphate buffer, and combinations thereof. In an example of the sinonasal treatment formulation, the salt includes a combination of sodium chloride and sodium bicarbonate.

When the nitric oxide generating formulation is the catheter lock formulation, any salt that provides physiological osmolarity and does not promote clotting may be used. Examples of suitable salts for the catheter lock formulation are selected from the group consisting of sodium chloride, sodium bicarbonate, a sodium phosphate buffer, a potassium phosphate buffer, and combinations thereof.

The additive selected is capable of controlling or accelerating the rate of release of nitric oxide from the RSNO species after the RSNO powder, the salt, and the additive are dissolved into a liquid carrier (e.g., for a sinonasal rinse or the catheter lock solution) or are incorporated into an aerosol spray (e.g., for a nasal spray). The inclusion of the additive in the antimicrobial sinonasal treatment formulation enables control over the time profile of NO release within the sinus cavities, which may enhance the therapeutic benefit. The inclusion of the additive in the catheter lock solution formulation enables control over the time profile of NO release within and from the catheter, which may enhance antimicrobial activity. Examples of the additive include reduced glutathione (GSH), cysteine, ascorbic acid or ascorbate, copper ions, zinc ions, zinc-oxide particles, an organoselenium species, or combinations thereof. Examples of the organoselenium species are selected from the group consisting of selenocysteine and ebeselen. An example of a combination of additives is reduced glutathione and ascorbate.

The following are some examples of how the additive can control or accelerate the rate of release of nitric oxide from RSNO, and in particular, from GSNO. Glutathione can increase the NO release rate from GSNO via the formation of an initial N-hydroxysulfenamide species (e.g., GS-N(OH)-SG), which then converts to a radical GS⁻ that can react with another GSNO molecule to liberate NO and form the GSSG disulfide species. Cysteine is able to transnitrosate with GSNO to form CysNO, which releases NO much faster than GSNO. Ascorbic acid or ascorbate can readily oxidize to form smaller threose structures (3 carbon sugars). The spontaneous oxidation of ascorbate can be coupled with reduction of GSNO to liberate NO plus GSH. Further, the oxidation products of ascorbate, i.e., the smaller threose structures, are also reducing agents that can provide electron(s) to GSNO, and thus may also contribute to the direct reduction of the GSNO to NO. In an example, the ascorbic acid or ascorbate may be allowed to oxidize in a solution for up to 5 days, dried, and then incorporated into the nitric oxide generating formulation. An organoselenium species can catalyze NO generation from GSNO. Copper or zinc ions may be reduced to their +1 oxidation state by any trace free thiols that exist in the GSH formulation, and the Cu(I) or Zn(I) ions can then reduce the GSNO to NO and GSH.

Within the nitric oxide generating formulation, the molar ratio of the RSNO powder to the additive ranges from 1:10 (0.1) to 10:1 (10). In an example, the molar ratio of the RSNO powder to the additive is about 1:1.

The antimicrobial sinonasal treatment formulation (including the RSNO powder, the salt selected from the group consisting of sodium chloride, sodium bicarbonate, calcium chloride, a sodium phosphate buffer, a potassium phosphate buffer, and combinations thereof, and the additive) may also be prepared so that when a predetermined amount of water or another liquid carrier is added to dissolve the components and form a sinus rinse/sinonasal treatment solution, the concentration of the RSNO in the sinonasal treatment solution ranges from about 25 µM to about 10 mM. In an example, the concentration of the RSNO in the sinus rinse/sinonasal treatment solution is about 100 µM

The catheter lock solution formulation (including the RSNO powder, the salt selected from the group consisting of sodium chloride, sodium bicarbonate, a sodium phosphate buffer, a potassium phosphate buffer, and combinations thereof, and the additive) may be prepared so that when a predetermined amount of water or another liquid carrier is added to dissolve the components and form a catheter lock solution, the concentration of the RSNO ranges in the catheter lock solution ranges from about 500 µM to about 50 mM.

The antimicrobial sinonasal treatment formulation (including the RSNO powder, the salt selected from the group consisting of sodium chloride, sodium bicarbonate, calcium chloride, a sodium phosphate buffer, a potassium phosphate buffer, and combinations thereof, and the additive) may also include a metal ion chelator. The metal ion chelator may be used to control the levels of free copper ions and/or zinc ions that are present in different chemicals or in the reconstituting fluid/solution. As an example, Cu(II) ions can act as a catalyst for RSNO decomposition (where Cu(II) is reduced to Cu(I) by free thiols, and the Cu(I) reduces the RSNO to NO and RSH). The metal ion chelator may be used to eliminate these effects, if they are not desirable. Examples of the metal ion chelator include ethylenediaminetetraacetic acid (EDTA), ethylene glycol tetraacetic acid (EGTA), sodium citrate, and combinations thereof. The antimicrobial sinonasal treatment formulation may be prepared so that when water or another liquid carrier is added to form a sinus rinse, the concentration of the metal ion chelator ranges from about 1 µM to about 1 mM.

The catheter lock solution formulation (including the RSNO powder, the salt selected from the group consisting of sodium chloride, sodium bicarbonate, a sodium phosphate buffer, a potassium phosphate buffer, and combinations thereof, and the additive) may also include an anticoagulant. The anticoagulant may provide local anticoagulant activity at the tip of the catheter as the anticoagulant diffuses out of the catheter. Examples of suitable anticoagulants include heparin, EDTA, sodium citrate, and combinations thereof. The inclusion of heparin may also enhance the antithrombotic activity at the catheter tip. The catheter lock solution formulation may be prepared so that when water or another liquid carrier is added to form the catheter lock solution, the concentration of the anticoagulant is present in an effective amount. An example of an effective amount of heparin ranges from about 2 units/ml (U/ml) to about 100 U/ml. An example of an effective amount of EDTA or sodium citrate ranges from about 0.5 mM to about 25 mM.

In any of the examples disclosed herein, the RSNO powder, the salt, and the additive, may be formulated together in a single powder composition (which may be contained in a single packet, container, etc.) or as a single pellet, tablet, etc. In some instances, the other additives, such as the metal ion chelator for the antimicrobial sinonasal treatment formulation or the anticoagulant for the catheter lock solution formulation, may also be part of the single powder composition or the single pellet, tablet, etc. Alternatively, in any of the examples disclosed herein, the RSNO powder may be contained within a first container, and the salt and the additive may be contained in a second container that is separate from the first container. The contents of each container may be added to the liquid carrier to form an example of the solution disclosed herein. Still further, in any of the examples disclosed herein, each of the RSNO powder, the salt, and the additive may be contained in respective and separate first, second and third containers. In this example, the contents of each container may be added to the liquid carrier to form an example of the solutions disclosed herein.

Still further, in any of the examples disclosed herein, the RSNO powder, the salt, and the additive, may be formulated together in the liquid carrier. As such, in some examples, the sinonasal treatment formulation (including the RSNO powder, the salt selected from the group consisting of sodium chloride, sodium bicarbonate, calcium chloride, a sodium phosphate buffer, a potassium phosphate buffer, and combinations thereof, and the additive) also includes the liquid carrier. In these examples, the liquid carrier may be selected from the group consisting of water, a saline solution, a saline solution with sodium bicarbonate (NaHCO₃), a phosphate buffered saline solution (PBS), an aqueous solution with calcium chloride (CaCl₂), and an aqueous solution with a sodium phosphate buffer or with a potassium phosphate buffer. In other examples, the catheter lock solution formulation (including the RSNO powder, the salt selected from the group consisting of sodium chloride, sodium bicarbonate, a sodium phosphate buffer, a potassium phosphate buffer, and combinations thereof, and the additive) also includes the liquid carrier. In these examples, the liquid carrier may be selected from the group consisting of water, a saline solution, a saline solution with sodium bicarbonate (NaHCO₃), a phosphate buffered saline solution (PBS), and an aqueous solution with a sodium phosphate buffer or with a potassium phosphate buffer.

The nitric oxide generating formulation may be included in a nitric oxide generating kit. The NO generating kit may be an antimicrobial sinonasal treatment kit or a catheter lock solution kit. In the NO generating kit, the RSNO may be part of a first powder composition, the additive may be part of a second powder composition, and the salt may be part of either the first or second powder composition, or may be in its own separate (third) powder composition. The NO generating kit also includes a container for dissolving the first and second powder compositions (one of which includes the salt), or the first, second, and third powder compositions into a liquid carrier.

In some examples of the kit, all of the nitric oxide generating formulation components are included in a single powder composition (which is a dry mixture of the listed components or a pellet, tablet, etc. containing the listed components). In other words, the previously mentioned first and second or first, second, and third powder compositions are combined together to form the single powder composition or a single pellet, tablet, etc. Within the kit, the single powder composition may be contained in a pouch, ampule, vial, or other suitable container, or may be compressed into a pellet, tablet, or the like that can be dissolved in a given volume of the liquid phase/liquid carrier. In an example of the sinonasal treatment kit, the RSNO powder, the salt selected from the group consisting of sodium chloride, sodium bicarbonate, calcium chloride, a sodium phosphate buffer, a potassium phosphate buffer, and combinations thereof, and the additive, with or without the metal ion chelator, are combined together into a single dry pellet or powder. In an example of the catheter lock solution kit, the RSNO powder, the salt selected from the group consisting of sodium chloride, sodium bicarbonate, a sodium phosphate buffer, a potassium phosphate buffer, and combinations thereof, and the additive, with or without the anticoagulant, are combined together into a single dry pellet or powder.

In other examples, the nitric oxide generating formulation includes the first powder composition and the second powder composition as separate compositions, one of which includes the salt. In still other examples, the nitric oxide generating formulation includes the first powder composition, the second powder composition, and the third powder composition as separate compositions. Within the kit, the separate powder compositions are contained in separate pouches, packets, or other suitable containers, or the separate powder compositions are respectively compressed into two or three different pellets, tablets, etc.

The nitric oxide generating kit may also include a container (of any type) for dissolving the powder composition(s) in a liquid carrier. Examples of the container for a sinonasal treatment kit include a nasal flush bottle or a neti-pot. Examples of the container for a catheter treatment kit include an ampule, or vial, or a syringe.

Some examples of the nitric oxide generating formulation disclosed herein may be added to a predetermined amount of the liquid carrier to form a sinus rinse or sinonasal treatment solution. The resulting sinus rinse/sinonasal treatment solution has a pH ranging from 4 to 10. The sinus rinse/sinonasal treatment solution may be prepared by a user and used immediately.

In the examples of the sinus rinse/sinonasal treatment solution, the liquid carrier may be any suitable liquid that is capable of being introduced into the upper airways of a human being through the nasal cavity. The liquid carrier may be water, a saline solution, a saline solution with sodium bicarbonate (NaHCO₃), a phosphate buffered saline solution (PBS), an aqueous solution with calcium chloride (CaCl₂), or an aqueous solution with a sodium phosphate buffer or a potassium phosphate buffer. It is to be understood that the salt that is present in the liquid carrier may be introduced when the nitric oxide generating formulation (including the salt) is dissolved therein. For example, sodium bicarbonate and sodium chloride may be included in the nitric oxide generating formulation that is added to water to form the sinus rinse/sinonasal treatment solution, or may be a separate packet that is added to the water in addition to the RSNO and additive components. In this particular example, when the salts are dissolved in water, the solution that is formed is a sodium bicarbonate/sodium chloride solution. In an example, this solution includes about 0.16 M sodium chloride and about 0.065 M sodium bicarbonate.

The examples of the nitric oxide generating formulation disclosed herein may also be added to an aerosol to form a nasal spray. In this example, an aerosol spray container can be filled with water and then the dry powder nasal formulation may be added to this reservoir to create the RSNO-containing solution that can be administered as an aerosol spray up into the nose and sinus cavities. The concentration of the RSNO in the aerosol spray may be equivalent to those used in the sinus rinse solution/sinonasal treatment solution approach. In some examples, the concentration of the RSNO in the aerosol spray may be higher than those used in the sinus rinse solution approach.

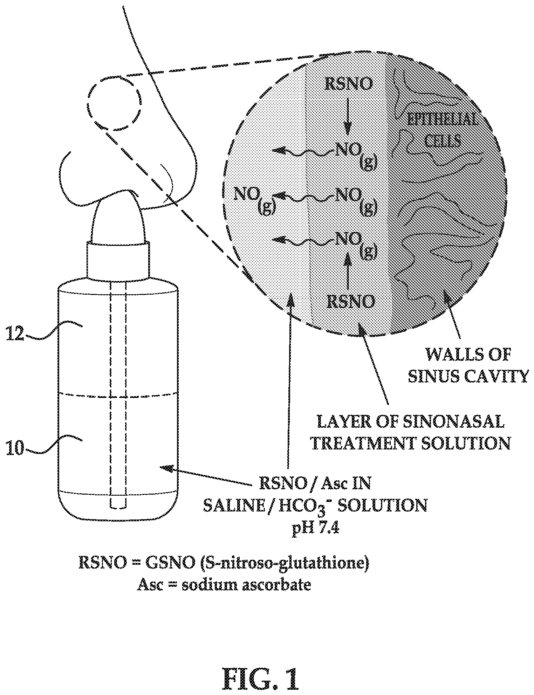

In the examples disclosed herein, the nitric oxide generating formulation or the kit may be used in a method for enhancing nasal nitric oxide (NO) levels. An example of this is shown in Fig. 1. The method involves introducing, via a nasal/sinus rinse 10 or aerosol spray (contained in a suitable container 12), the *S*-nitrosothiol (e.g., *S*-nitrosoglutathione (GSNO)) and the additive into a nasal cavity.

When the nasal/sinus rinse is utilized, it may be desirable to freshly prepare the rinse 10 in the container 12 by adding the nitric oxide generating formulation to the liquid carrier and stirring or shaking so that the powder(s), tablet(s), pellet(s), etc. dissolve, and then introducing the rinse into the nasal cavity. When the aerosol spray is utilized, the aerosol container may be configured to freshly prepare the aerosol solution by introducing the powder(s), tablet(s), pellet(s), etc. into the liquid carrier and aerosolizing the solution as it is being dispensed into the nasal cavity.

When the sinus rinse/sinonasal treatment solution (including an example of the nitric oxide generating formulation in a saline/HCO₃ solution) is introduced into the nasal cavity, the RSNO and additive (where RSNO = GSNO in Fig. 1) is also introduced into the nasal cavity. Upon introducing the sinus rinse into a nostril, a significant volume of the nitric oxide generating formulation is retained as a layer of sinonasal treatment solution (as shown in Fig. 1) within pockets along the surface of the sinus/nasal mucosal tissue that line the inner walls of sinus cavities. The RSNO in solution spontaneously decomposes at a controlled rate (which may be controlled by the additive that is used) to emit NO. This increases the levels of NO in the airwaves, as well as at the surface of the sinus/nasal mucosal tissue, on which biofilm may reside.

The increased levels of NO that are generated will be therapeutic, and will be sufficient to help kill bacteria and viruses, disrupt bacterial biofilm formation, disperse or prevent microbial biofilm formation (e.g., disperse antibiotic resistant biofilms), reduce inflammation, and increase ciliary beat frequency, thus improving mucociliary clearance. Elevated NO production may be observed nearly immediately and for an extended period (e.g., up to about 4-6 hours). The nasal formulation disclosed herein may be used to fight an infection. Furthermore, daily use (e.g., one or two times per day) of the nasal formulation disclosed herein may help maintain NO levels equivalent to those of healthy individuals (i.e., may help in keeping sinuses healthy in a manner that is consistent with how the human body fights infection), which, in turn, may prevent formation of bacterial biofilm and CRS disease perpetuation.

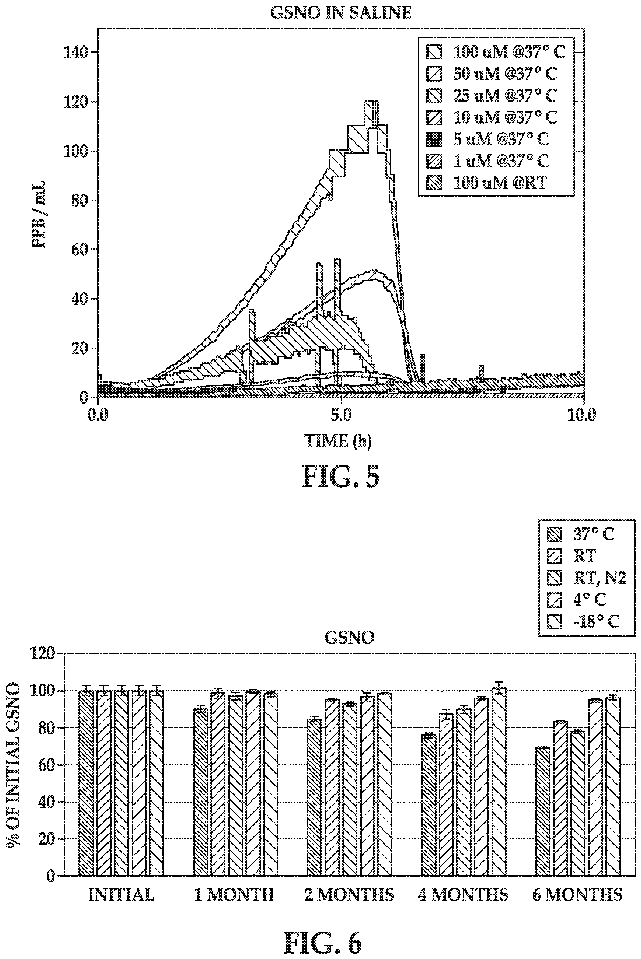

The risk of producing too much NO (toxic levels) with the examples disclosed herein is unlikely. For example, typical sinus cavities have a volume of about 60 ml. Therefore, if 10% of a 250 ml volume of sinus rinse remains in the sinuses (along sinus cavity walls and within crevices, etc.), and the rinse contains 10 µM of GSNO, the highest level of gas phase NO possible in the sinonasal cavities could be about 80 ppmv, if all the NO is emitted immediately (which is not possible given the slow release of NO from the GSNO over several hours; see, e.g., Figs. 3 and 6). In the examples disclosed herein, the levels of gas-phase NO within the sinus cavities as a result of introducing the nitric oxide generating formulation via a rinse or aerosol spray will be much lower and closer to targeted normal 200 - 500 ppbv levels.

Other examples of the nitric oxide generating formulation disclosed herein may be added to a predetermined amount of the liquid carrier to form a catheter lock solution. The resulting catheter lock solution has a pH ranging from 4 to 10. The catheter lock solution may be prepared by a user and used immediately.

The liquid carrier used to form the catheter lock solution may be any suitable liquid that is capable of being introduced into a catheter and that will not promote blood clotting in a patient. In these examples, the liquid carrier may be water, a saline solution, a saline solution with sodium bicarbonate (NaHCO₃), a phosphate buffered saline solution (PBS), or an aqueous solution with a sodium phosphate buffer or a potassium phosphate buffer. It is to be understood that the salt that is present in the liquid carrier may be introduced when the catheter lock solution formulation (including the salt) is dissolved therein. For example, sodium bicarbonate and sodium chloride may be included in the catheter lock solution formulation that is added to water to form the catheter lock solution, or may be a separate packet that is added to the water in addition to the RSNO and additive components.

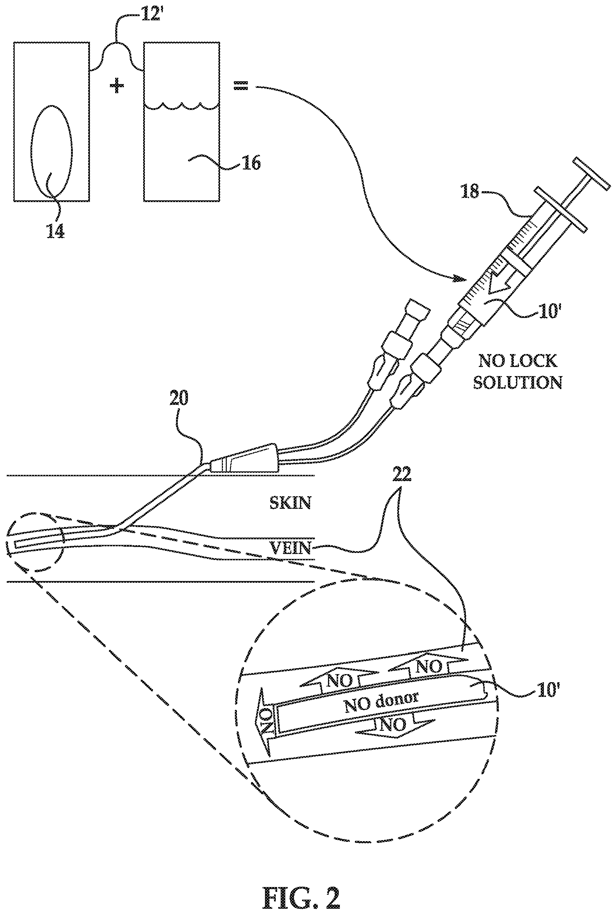

In the examples disclosed herein, the catheter lock solution formulation or the kit may be used in a method for enhancing antimicrobial nitric oxide (NO) levels both inside and outside of a catheter that is injected into a patient. An example of this is shown in Fig. 2.

The method involves preparing the catheter lock solution 10' by adding the catheter lock solution formulation 14 to the liquid carrier 16 in a container 12', and stirring or shaking so that the powder(s), tablet(s), pellet(s), etc. of the formulation 14 dissolve in the liquid carrier 16. In an example, from about 2 mL to about 3 mL of pure water is added to a tablet of the RSNO, the salt, and the additive to form the catheter lock solution 10'. In other examples, the different powder compositions may be added to the liquid carrier 16 in the container 12', and stirred or otherwise mixed to dissolve the compositions and form the catheter lock solution 10'.

The method then involves introducing the catheter lock solution 10' into a lumen of the catheter 20 (which is positioned in a blood vessel 22 of a patient). A syringe 18 (or other suitable instrument) may be used to take up the catheter lock solution 10' (from the container 12') and to introduce the catheter lock solution 10' into the lumen.

The reconstituted catheter lock solution 10' releases low levels of NO for an extended time period. At least the distal end of the catheter lumen releases the generated NO. The NO at the distal end inhibits local platelet activity, thrombus formation, and distal occlusion. When the walls of the lumen and the catheter 20 are permeable to nitric oxide (as shown in Fig. 2), the NO may also partition favorably out of the lumen and the catheter walls. Examples of NO permeable materials that may be used for the lumen and the catheter 20 include silicone rubber (SR), polyurethane (PU), copolymers of SR and PU, copolymers of PU and polycarbonate, and the other NO permeable materials. When the walls of the lumen and the catheter 20 are permeable to nitric oxide, the NO may be emitted over the entire outer surface of the catheter 20, which at least substantially prevents bacteria adhesion, biofilm formation, and clotting on the outer catheter surface. For example, the increased levels of NO that are generated will be therapeutic, and will be sufficient to help kill bacteria and viruses, disrupt bacterial biofilm formation, and disperse or prevent microbial biofilm formation (e.g., disperse antibiotic resistant biofilms). At the same time, even higher levels of NO will exist in the lock solution within the lumen of the catheter 20, and this will prevent infection within that lumen and other lumens, if the device 20 is a multilumen catheter. Moreover, when the anticoagulant is included in the nitric oxide generating formulation 14 that is used to form the catheter lock solution 10', the antithrombotic effects of the catheter lock solution 10' at the distal end of the catheter 20 may be further improved.

In the examples disclosed herein, adding the dry nitric oxide generating formulation to the liquid carrier forms an active solution. Reconstituting the nitric oxide generating formulation in the liquid carrier immediately before use in the nasal cavity or in the catheter may be desirable because the freshly prepared solutions are more stable than solutions in which the RSNO has been sitting for an extended period (e.g., a few days).

To further illustrate the present disclosure, examples are given herein. It is to be understood that these examples are provided for illustrative purposes and are not to be construed as limiting the scope of the present disclosure.

### EXAMPLE 1

Two different solutions were prepared, one with 10 µM of GSNO and one with 25 µM of GSNO, in phosphate buffered saline at physiological pH 7.4. These solutions did not include an additive as disclosed herein.

The NO release profile of the two solutions was measured via chemiluminescence detection at 37°C, and the results are shown in Fig. 3. As depicted, both solutions continuously generated NO for more than 7 hours, and the solution containing the higher concentration of GSNO generated higher levels of NO. It should be noted that the levels of NO observed for these solutions in this Example, generally in the 5 ppbv to 40 ppbv range, are diluted significantly via the continuous flush of the test solution with a nitrogen stream that sweeps the released NO into the chemiluminescence NO analyzer, compared to gas levels that would be actually observed in the sinus cavities.

Two additional solutions were prepared, one with 100 µM of GSNO and one with 100 µM of GSNO and 100 µM of ascorbate (i.e., one example of the additive disclosed herein), in 0.16 M NaCl/0.03 M NaHCO₃.

The NO release of the two additional solutions was measured via chemiluminescence, and the results are shown in Fig. 4. As depicted, the inclusion of ascorbate provided an enhanced NO release profile, with all of the NO release within a 2-3 hour window. It should be noted that the additional solutions tested were purged with nitrogen to sweep the NO released from GSNO into the chemiluminescence NO gas phase analyzer, and hence the rate of release may be faster than what may be observed when the formulation is used as a sinus rinse or aerosol spray.

### EXAMPLE 2

### Formulations

In this Example, several nasal formulations were prepared using previously synthesized S-nitrosoglutathione (GSNO), alone or in combination with reduced L-glutathione (GSH, >98%) or L-ascorbic acid (Asc). Some of the formulations also included ethylenediamintetraacetic acid (EDTA) and/or saline salts (NaCl and NaHCO₃). The constituents were mixed as dry powders and were stored in 4 mL amber vials either in the freezer (-18°C), at 4°C, at room temperature (RT, from about 20°C to about 25°C), or at 37°C.

The various dry powder nasal formulations included GSNO, GSNO and GSH (1:1 molar ratio), GSNO, GSH, and EDTA (1:1:0.1 molar ratio), GSNO:Asc (1:1 molar ratio), and GSNO:Asc:EDTA (1:1:0.1 molar ratio).

A dry powder nasal formulation of GSNO and EDTA was prepared with the saline salts, and a dry powder nasal formulation of GSNO and GSH was prepared with the saline salts. Both of these formulations were prepared so that when diluted to 200 mL (with water), the final concentration of GSNO was 100 µM.

### NO Release Rate

The NO release rate of some of the solution formulations was measured by chemiluminescent nitric oxide analyzer (NOA) at room temperature and at 37°C in an amber NOA cell, while purging with 50 mL/min nitrogen through a glass pipette. The solution formulations were freshly prepared (initial data) (i.e., the dry powder nasal formulation was dissolved in 10 mL of saline solution (NaCl and NaHCO₃ in water) immediately before measurements), or were prepared from stored dry power aliquots that were freshly dissolved in 10 mL of saline solution (NaCl and NaHCO₃ in water) immediately before measurements. The NO release rate was tested for one or more of the following time periods: 0 (freshly prepared), 1 month, 2 months, 4 months, and 6 months.

The NOA was a Sievers NOA. Studying the release kinetics included analyzing the overall shape of the transient curve, half-life, and total NO released compared to theoretical GSNO content (%). Primarily, the shape of the graph was considered most qualitative, as different machines yield different PPB/mL values due to differing constants. If necessary, the GSNO quantification in each formulation was tested by a copper/cysteine method to release all of the NO from the GSNO very rapidly.

### Stability Measurements

The stability of some of the dry powder formulations was tested via UV/Vis analysis and/or via the Sievers nitric oxide (NO) analyzer (as previously described).

For the UV/Vis analysis, at each time interval (0 (freshly prepared), 1 month, 2 months, 4 months, and 6 months), three samples (3-5 mg each) from the dry powder formulations (at each temperature, resulting in 12 vials per formulation) were put into 4 mL amber vials. The respective formulations were then diluted in 4 mL PBS + 0.1 mM EDTA (PBSE) solution and mixed for 30 seconds, and 1 mL of the diluted formulation was injected into 3 mL PBSE in a disposable UV/VIS cuvette. The absorbance spectrum was measured typically between 300-600 nm after measuring the PBSE spectrum as blank.

GSNO was quantified by absorbance measured at 334 nm. These values were then compared to a calculated value of the percentage of initial or theoretical GSNO content of each formulation. As discussed below, GSH, ascorbate, and EDTA do not have absorbance at 334 nm, therefore this wavelength was used for selective quantification of GSNO in each formulation.

### GSH Stability

To detect GSH stability, Ellman's assay was used. This assay detects the amount of free thiol groups in a compound after reacting the compound with 5,5'-dithiobis-(2-nitrobenzoic acid) (DTNB) to produce a yellow colored product with absorbance at 412 nm. This indicates that GSH has no interfering absorbance at 334 nm.

### Ascorbic Acid Stability

Ascorbate (i.e., vitamin C) is known to be stable for a very long time in dry conditions. As will be discussed in more detail below, the UV/Vis analysis for GSNO mixed with ascorbic acid are similar for freshly prepared samples and samples prepared after the dry powder formulation had been stored for several months. These results indicate that the ascorbic acid is relatively stable.

### EDTA Stability

EDTA is also known to be stable for a very long time in dry conditions. As will be discussed in more detail below, the NO release patterns for GSNO mixed with GSH or ascorbic acid and EDTA are similar for freshly prepared samples and samples prepared after the dry powder formulation had been stored for several months. These results indicate that the EDTA is stable.

### GSNO Results

Fig. 5 depicts the NO release kinetics of various GSNO formulations in saline at 37°C and at room temperature. The various dry powder mixtures were prepared, and the solutions were prepared right before taking the measurements of NO release. For the 37°C solutions, the GSNO concentrations were 1 µM, 5 µM, 10 µM, 25 µM, 50 µM, and 100 µM. For the room temperature solution, the GSNO concentration was 100 µM The results in Fig. 5 show that there is a rather long lag time in the release of NO from the GSNO without any additive, peaking for all concentrations in the 5-6 hour time period. Further, as shown, the amount of NO release is directly related to the GSNO concentration in the test solution.

Fig. 6 is a bar graph depicting the stability, in terms of % of initial GSNO versus time (as measured by UV/Vis spectroscopy), of GSNO formulations when freshly prepared (i.e., initial) and when stored as dry powder at different temperatures for different time periods. For the results shown in Fig. 6, all of the dry powder formulations contained 100 µM GSNO. As mentioned above, for UV/Vis, the respective dry powder formulations were diluted in 4 mL PBS + 0.1 mM EDTA (PBSE) solution and mixed for 30 seconds, and 1 mL of the diluted formulation was injected into 3 mL PBSE in a disposable UV/VIS cuvette. The stability tests for the GSNO formulations have been completed for a six month period of time. The UV/VIS analysis showed no significant decrease in the stability, although the 37°C aliquot showed the largest decrease in stability with 67% remaining after 6 months (compared to 100% in the freshly prepared/initial sample). The headspace within the vials used to store the GSNO formulations was room air, which had some moisture. Hence, the true shelf life stability of the GSNO formulations in a completely dry headspace atmosphere is likely to be much better.

### GSNO:GSH (1:1) Results

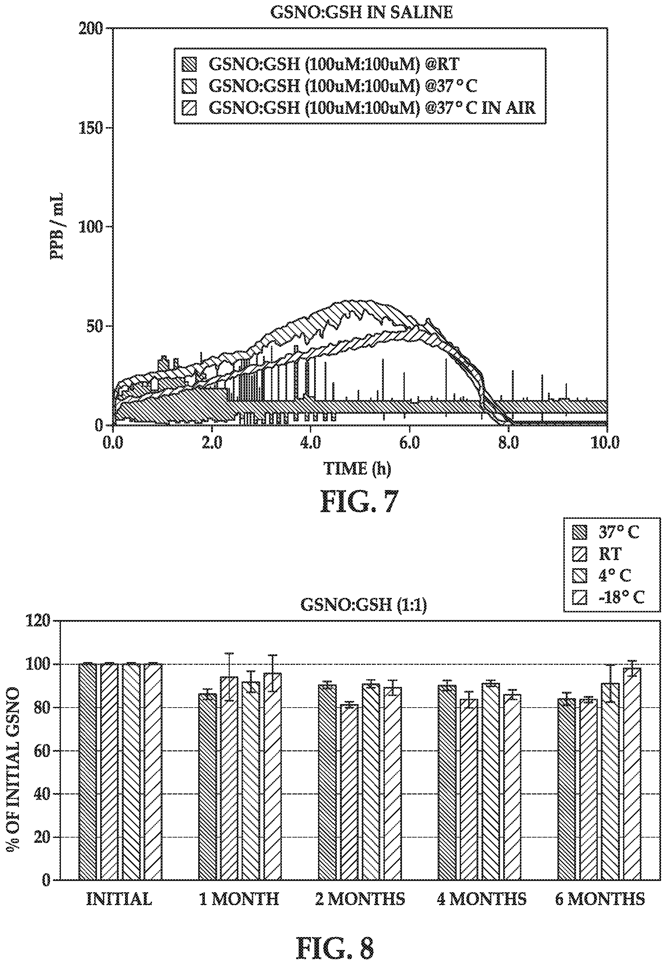

Fig. 7 is a graph depicting the NO release kinetics of GSNO:glutathione (GSH) (1:1) in saline at 37°C, at room temperature, and at 37°C in the presence of oxygen (air). Each of these solutions was freshly prepared with the dry powder formulation that has not been stored. For each solution, the GSNO and GSH concentrations were each 100 µM. The results in Fig. 7 show that at higher temperature the rate of NO release is increased. Further, the NO release rate is slightly enhanced, especially at the very beginning compared to when solutions are made up without GSH present (see Fig. 5).

Fig. 8 is a bar graph depicting the stability, in terms of % of GSNO versus time (as measured by UV/Vis spectroscopy), of GSNO:GSH (1:1) formulations when freshly prepared (i.e., initial) and when stored as dry powder at different temperatures for different time periods. For the results shown in Fig. 8, all of the dry powder formulations included 100 µM GSNO: 100 µM GSH. As mentioned above, for UV/Vis, the respective dry powder formulations were diluted in 4 mL PBS + 0.1 mM EDTA (PBSE) solution and mixed for 30 seconds, and 1 mL of the diluted formulation was injected into 3 mL PBSE in a disposable UV/VIS cuvette. The stability tests for the GSNO:GSH formulations have been completed for a six month period of time. The UV/VIS analysis showed no significant decrease in the stability over the six month period of time. The headspace within the vials used to store the GSNO formulations was room air, which had some moisture. Hence, the true shelf life stability of the GSNO formulations in a completely dry headspace atmosphere is likely to be much better.

### GSNO:GSH:EDTA (1:1:0.1) Results

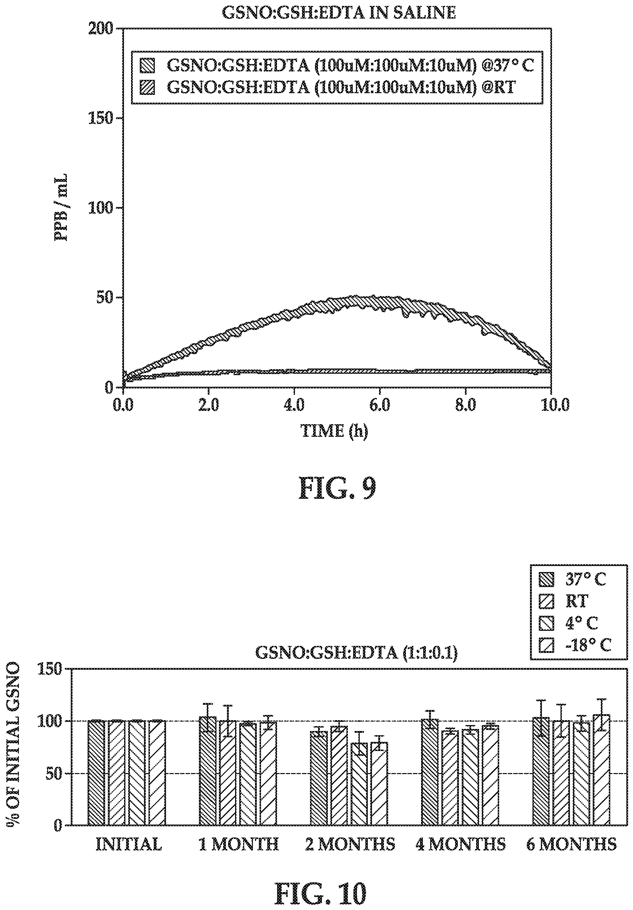

Fig. 9 is a graph depicting the NO release kinetics of GSNO:GSH: ethylenediaminetetraacetic acid (EDTA) (1:1:0.1) in saline at 37°C and at room temperature. Each of these solutions was freshly prepared with the dry powder formulation that had not been stored. For each solution, the GSNO and GSH concentrations were each 100 µM. The results in Fig. 9 show that in the presence of EDTA, the rate of release of NO is slowed somewhat due to EDTA chelating trace metal ion contaminants in the chemicals used to prepare the solutions, and that these trace metal ions, especially copper ions, can act as catalysts for GSNO decomposition.

Fig. 10 is a bar graph depicting the stability, in terms of % of GSNO versus time (as measured by UV/Vis spectroscopy), of GSNO:GSH:EDTA (1:1:0.1) formulations when freshly prepared (i.e., initial) and when stored as dry powders at different temperatures for different time periods. For the results shown in Fig. 10, all of the dry powder formulations included 100 µM GSNO: 100 µM GSH: 10 µM EDTA. As mentioned above, for UV/Vis, the respective dry powder formulations were diluted in 4 mL PBS + 0.1 mM EDTA (PBSE) solution and mixed for 30 seconds, and 1 mL of the diluted formulation was injected into 3 mL PBSE in a disposable UV/VIS cuvette. The stability tests for the GSNO:GSH formulations have been completed for a six month period of time. The UV/VIS analysis showed a slight decrease in the stability at the two month period of time for the samples stored at 4°C and -18°C, but showed overall stability over the six month period of time. The headspace within the vials used to store the GSNO formulations was room air, which had some moisture. Hence, the true shelf life stability of the GSNO formulations in a completely dry headspace atmosphere is likely to be much better.

### GSNO:Asc Results

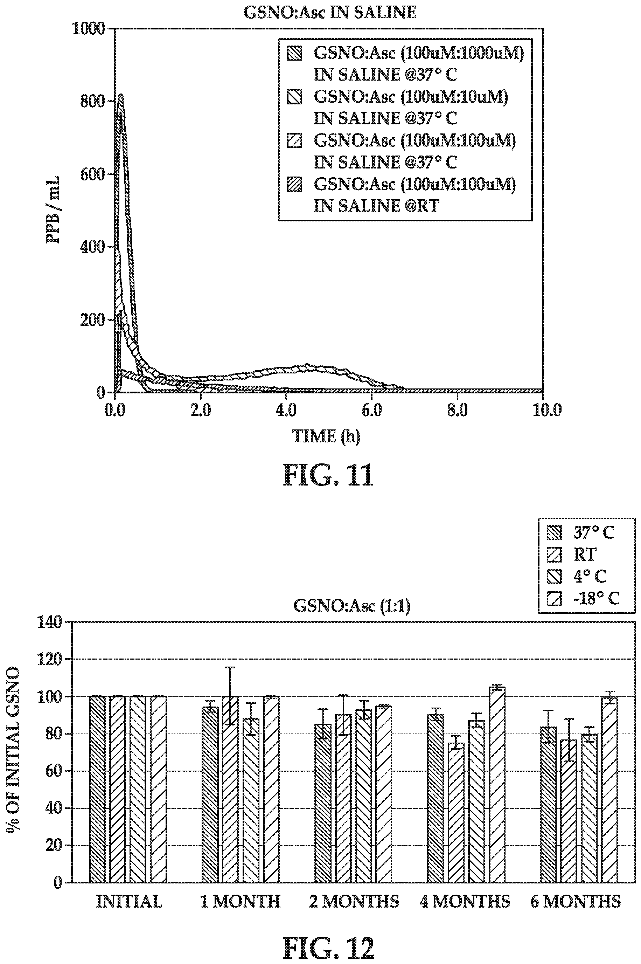

Fig. 11 is a graph depicting the NO release kinetics of GSNO:Ascorbic acid (Asc) at different ratios in saline at 37°C and of GSNO:Asc (1:1) in saline at room temperature. Each of these solutions was freshly prepared with the dry powder formulation that had not been stored. For the 37°C solutions, the GSNO:Asc concentrations were 100 µM:1000 µM, 100 µM:10 µM, and 100 µM: 100 µM. The results in Fig. 11 show that at higher concentrations of ascorbate, a large spike is seen before the two-hour period of time, demonstrating that the presence of ascorbate greatly speeds the release of NO from GSNO. The 100 µM:100 µM concentration in saline at 37°C still produces an initial spike but takes longer to reach the baseline. The lowest concentration of ascorbate additive, 10 µM, has very little effect on the NO release rate, with maximum production at the 5-6 hour time point, similar to just GSNO in saline with no other additives.

Fig. 12 is a bar graph depicting the stability, in terms of % of GSNO versus time (as measured by UV/Vis spectroscopy), of GSNO:Asc (1:1) formulations when freshly prepared (i.e., initial) and when stored as dry powders at different temperatures for different time periods. For the results shown in Fig. 12, all of the dry powder formulations included 100 µM GSNO: 100 µM Asc. As mentioned above, for UV/Vis, the respective dry powder formulations were diluted in 4 mL PBS + 0.1 mM EDTA (PBSE) solution and mixed for 30 seconds, and 1 mL of the diluted formulation was injected into 3 mL PBSE in a disposable UV/VIS cuvette. The stability tests for the GSNO:Asc formulations have been completed for a six month period of time. The UV/VIS analysis showed good stability for all of the samples, although the stability of the sample stored at room temperature decreased more significantly at four months. The headspace within the vials used to store the GSNO formulations was room air, which had some moisture. Hence, the true shelf life stability of the GSNO formulations in a completely dry headspace atmosphere is likely to be much better.

### GSNO:Asc:EDTA (1:1:0.1) Results

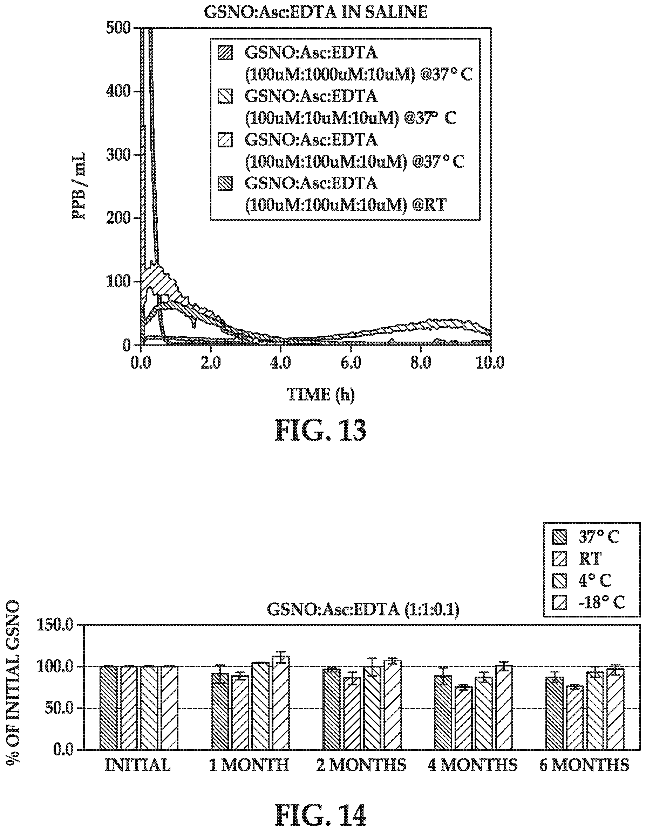

Fig. 13 is a graph depicting the NO release kinetics of GSNO:Asc:EDTA at different ratios in saline at 37°C and of GSNO:Asc:EDTA (1:1:0.1) in saline at room temperature. Each of these solutions was freshly prepared with the dry powder formulation that had not been stored. As already seen with the GSNO:Asc formulation, it is important to control the concentration of ascorbate to avoid a large initial spike and fast decline in NO release ppb/mL. The presence of EDTA decreased the free trace copper ion level (or other trace metal ions) and lengthened this decline, making the NO release last closer to 5 hours, as opposed to 3-4 hours without the EDTA.

Fig. 14 is a bar graph depicting the stability, in terms of % of GSNO versus time (as measured by UV/Vis spectroscopy), of GSNO:Asc:EDTA (1:1:0.1) formulations when freshly prepared and when stored as dry powders at different temperatures for different time periods. For the results shown in Fig. 14, all of the dry powder formulations included 100 µM GSNO: 100 µM Asc:10 µM EDTA. As mentioned above, for UV/Vis, the respective dry powder formulations were diluted in 4 mL PBS + 0.1 mM EDTA (PBSE) solution and mixed for 30 seconds, and 1 mL of the diluted formulation was injected into 3 mL PBSE in a disposable UV/VIS cuvette. The stability tests for the GSNO:Asc:EDTA formulations have been completed for a six month period of time. The UV/VIS analysis showed good stability for all of the samples. The data for the room temperature sample indicates a decrease in stability at 4 months. However, this decrease may be due to a weighing error when filling the vials and/or to the dry powder formulation not being homogeneous when broken out into the different storage temperature batches. Moreover, headspace within the vials used to store the GSNO formulations was room air, which had some moisture. Hence, the true shelf life stability of the GSNO formulations in a completely dry headspace atmosphere is likely to be much better.

### Formulations Combined with Saline

Two formulations (GSNO:EDTA with saline and GSNO:GSH with saline) have been formed to test the packaging of the sinus rinse. The first month time period passed and, at this time, no decomposition had been detected.

### EXAMPLE 3

### 3A - Effect of GSNO:Asc (1:1) on S. aureus biofilm disruption

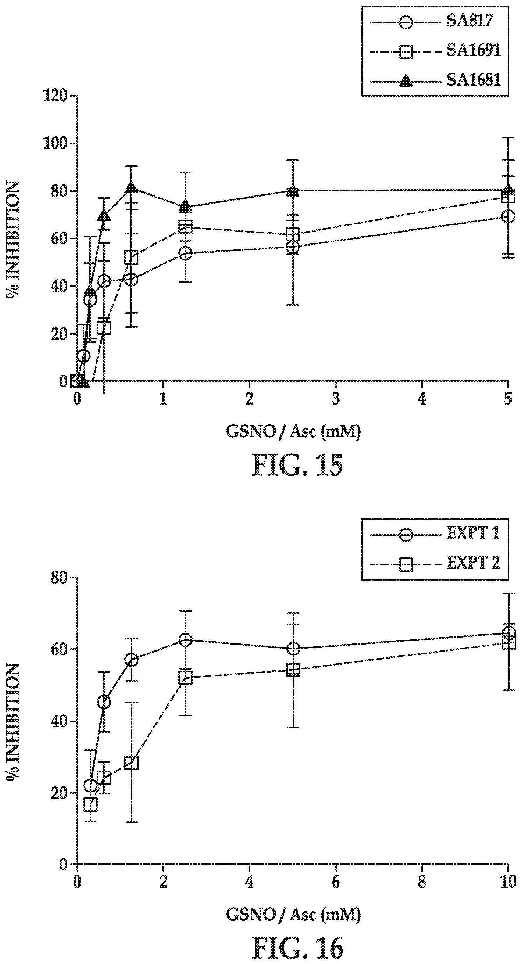

Three strains (SA817, SA1691, and SA 1681) *of S. aureus* (1 x 10⁴ colony-forming units/100 µl or CFU/100 µl) were plated, respectively, in 96 well microtiter plates and incubated overnight. Planktonic bacteria were removed and washed with saline once. Various concentrations of GSNO:ascorbic acid (1:1 or equimolar) were diluted in saline, and the saline solution (0.12 M, 100 µl) was added to the bacteria biofilms remaining on the surfaces of the wells. The bacteria were incubated for 4 hours at room temperature (RT) and then the biofilm density was measured by staining with crystal violet. The experiment was performed 3 times with 6 replicates for each strain (i.e., N=6 for each data point in Fig. 15, plus or minus standard deviation). The experimental results are shown in Fig. 15. In particular, Fig. 15 shows the effect of GSNO/Asc on the 3 different *S. aureus* isolates (SA817, SA1691, and SA 1681). As depicted in Fig. 15, significant reduction of the biofilm density was observed as the GSNO/Asc concentrations were increased between 0 mM and 1 mM. A single exposure of the bacterial biofilm to the GSNO/Asc solution for 4 hours reduced biofilm density *of S. aureus* strains by nearly 50% at ca. 0.3 mM concentration.

### 3B - Effect of GSNO:Asc (1:1) on killing of P. aeruginosa biofilm growth on CF cultures

*P. aeruginosa* (1 x 10⁴ colony-forming units/100 µl or CFU/100 µl) was plated in a 96 well microtiter plate and incubated overnight. Planktonic bacteria were removed and washed with saline once. Various concentrations of GSNO:ascorbic acid (1:1 or equimolar) were diluted in saline, and the saline solution (0.12 M, 30 µl) was added to the bacteria biofilms remaining in the wells. The bacteria were incubated for 4 hours at room temperature (RT) and then the biofilm density was measured by staining with crystal violet. The experiment was performed 2 times (EXPT 1 and EXPT 2 in Fig. 16) with 6 replicates (i.e., N= 6 for each data point in Fig. 16, plus or minus standard deviation). The experimental results are shown in Fig. 16. In particular, Fig. 16 shows the effect of GSNO/Asc on the *P. aeruginosa* isolates. As depicted in Fig. 16, significant reduction of the biofilm density was observed as the GSNO/Asc concentrations were increased between 0 mM and 2.5 mM. These results in Fig. 16 are similar to the results in Fig. 15, although somewhat higher doses of GSNO/Asc were required to maximize the disruption of preformed biofilm of the *P. aeruginosa* microbe. This may be related to the fact that *P. aeruginosa* is a nitrifying organism that has some NO reductase enzyme activity within. Further, the plateaus observed at high doses may be related to faster loss of NO to the atmosphere above the biofilms/wells from the thin layer of solution of GSNO/Asc applied to the surface of the biofilm. At higher concentrations, the rate of NO generation will increase, but this is offset by faster loss of the NO into the air above the films.

### 3C - Effect of GSNO:Asc (1:1) on killing of S. aureus biofilm growth on CF cultures

Progenitor (basal) airway epithelial cells isolated from cystic fibrosis patients were cultured at air/liquid interface to promote mucociliary differentiation resembling airway epithelium *in vivo.* These cultures will be referred to herein as CF airway epithleial cells.

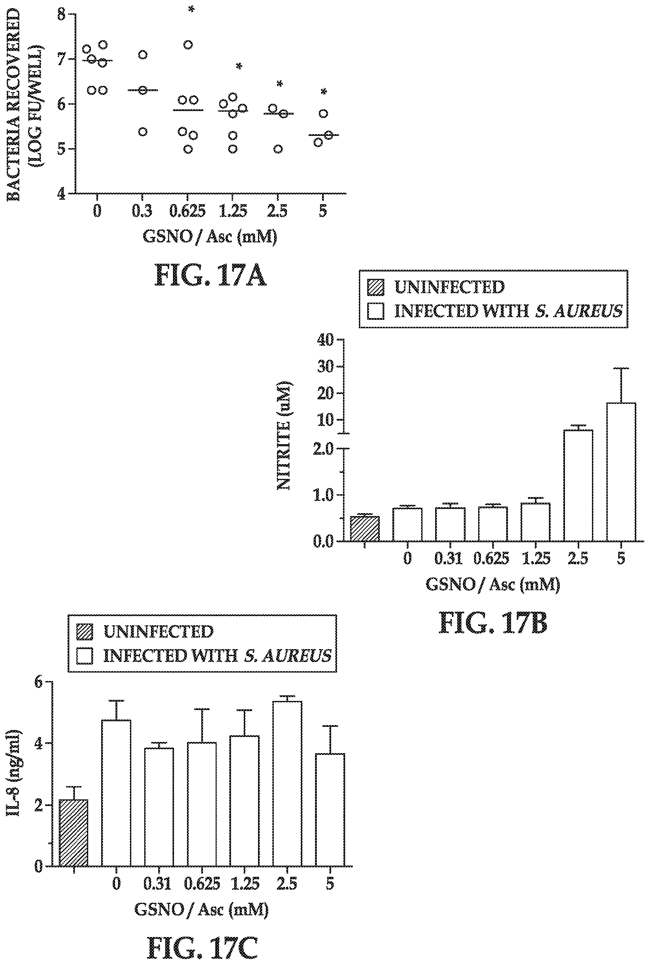

Apical surface of the CF airway epithelial cells was washed with PBS, infected apically with 10 µl *of S. aureus* (1 x 10⁵ CFU/well or at 0.01 multiplication of infection), and incubated for 24 hours. Control samples (for determination of IL-8 and nitrite analysis) were washed in a similar manner but were not infected with *S. aureus.* The basolateral medium was changed, the apical surface was washed with saline once, and then 30 µl of PBS containing various concentrations of GSNO/Asc (i.e., 0 mM, 0.3 mM, 0.625 mM, 1.25 mM, 2.5 mM, and 5 mM) was added to the apical surface and incubated for 4 hours. The apical surface was washed once with 0.2 ml of 0.15% bicarbonate in PBS. Washings were used for nitrite analysis (Fig. 17B). Cells were lysed in 1% Triton X-100 and serial dilutions of cell lysates were plated to determine the bacterial density (Fig. 17A). IL-8 protein in the basolateral medium was determined by ELISA (Fig. 17C). The data in Fig. 17A represents median and range and the data in Figs. 17B and 17C represent mean±standard deviation (S.D.) from 3 to 6 experiments. In Fig. 17A, * p<0.05, which is different from saline alone treated cells cultures. Overall, the results in Figs. 17A through 17C illustrate that GSNO/Asc at 0.625 mM reduces bacterial density by more than one log unit without inducing IL-8, a pro-inflammatory cytokine. Moreover, the GSNO/Asc treatment did not induce cytotoxicity or pro-inflammatory effects (as determined by IL-8 release) at doses up to 5 mM. Fig. 17B shows that as increasing levels of GSNO solution are employed, the levels of nitrite at the apical surface increase, as expected.

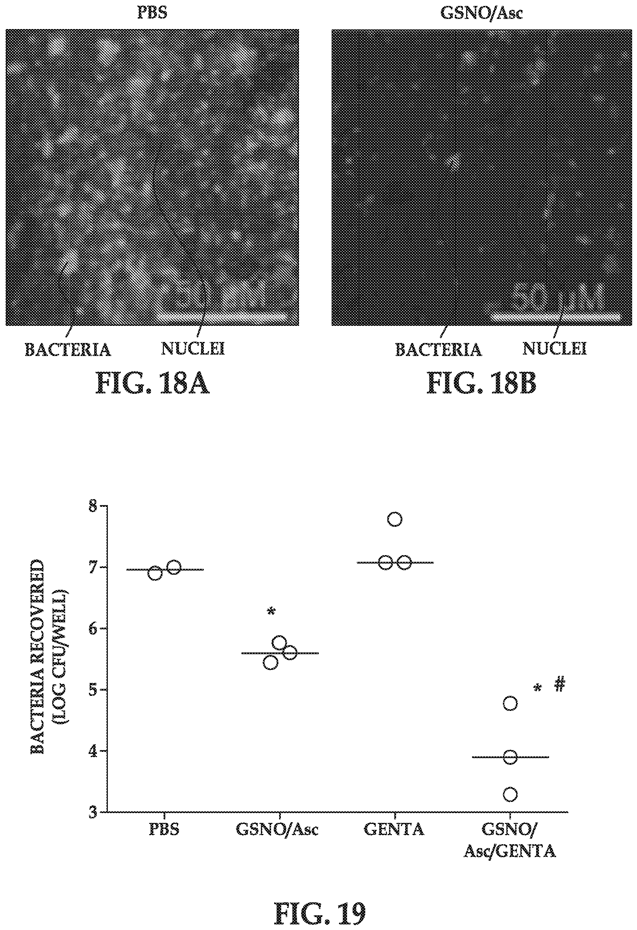

To confirm that reduction in live bacterial counts was associated with disruption of biofilm, confocal microscopy was performed. CF cell cultures with established *S. aureus* biofilm on the apical surface were treated with PBS or GSNO/Asc as previously described. Cultures were briefly washed and fixed in paraformaldehyde, blocked with 5% normal donkey serum, and then incubated with antibody to *S. aureus* overnight at 4°C. The unbound antibody was washed off, and the bound antibody was detected by a second antibody conjugated with Alexafluor-488 and the cells were counter stained with DAPI to detect nuclei. The cultures were mounted and visualized under confocal microscope. Compared to the PBS treated cultures, the GSNO/Asc treated cultures showed very few bacteria with visually no bacterial biofilm, indicating that GSNO/Asc disrupts bacterial biofilm in addition to killing bacteria. The results, which were originally in color and where green depicted the bacteria and blue depicted the nuclei, are shown in black and white in Figs. 18A and 18B, where lighter spots depict the bacteria and darker spot depict the nuclei. The PBS treated culture is shown in Fig. 18A, and the original image was predominately green. In contrast, the GSNO/Asc treated culture is shown in Fig. 18B, and the original image was predominately blue. The treatment with GSNO/Asc significantly reduced the density of bacterial biofilm growth on the apical surfaces of the mucociliary differentiated cell cultures.

### 3D - Synergistic Effect of GSNO:Asc (1:1) and gentamicin on killing of colonized S. aureus on CF airway epithelial cells

Apical surface of CF airway epithelial cells was washed with PBS, infected apically with 10 µl *of S. aureus* (2 x 10⁶ CFU/well), and incubated for 24 hours. The basolateral medium was changed, the apical surface was washed with saline once and treated apically with 30 µl of PBS, or saline containing 1.25 mM GSNO/Asc, or saline containing 50 µg of gentamicin, or saline containing a mixture of 1.25 mM GSNO/Asc and 50 µg of gentamicin. All cells were incubated for 4 hours. The apical surface was washed once with 0.2 ml of 0.15% bicarbonate in PBS and then with PBS. Cells were lysed in 1% Triton X-100 and serial dilutions of cell lysates were plated to determine the bacterial density. The results are shown in Fig. 19 (N=3). The data represent the median and range from 3 experiments. In Fig. 19, * p<0.05, which was different from PBS treated cultures; and # p<0.05, which was different from GSNO/Asc treated cultures. These results indicate that a single application of GSNO/Asc has a significant impact on the number of live *S. aureus* cells remaining on the surface (20-30 fold decrease). These results also indicate that the combination of gentamicin and GSNO/Asc is significantly more effective in killing bacteria than GSNO/Asc alone.

### 3E - Effect of GSH and Ascorbic Acid on killing of colonized S. aureus on CF airway epithelial cells

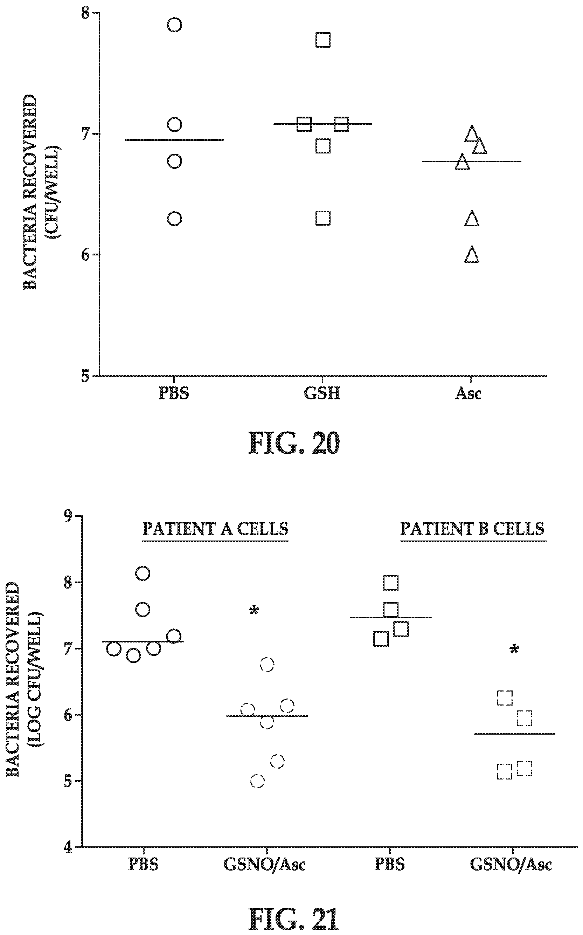

Apical surface of CF airway epithelial cells was washed with PBS, infected apically with 10 µl of *S. aureus* (2 x 10⁶ CFU/well), and incubated for 24 hours. The basolateral medium was changed, the apical surface was washed with saline once, and treated apically with 30 µl of PBS, or saline containing 1.25 mM GSH, or saline containing 1.25 mM Ascorbic acid. All cells were incubated for 4 hours. The apical surface was washed once with 0.2 ml of 0.15% bicarbonate in PBS and then with PBS. Cells were lysed in 1% Triton X-100 and serial dilutions of cell lysates were plated to determine the bacterial density. The results are shown in Fig. 20. The data represent the median and range from 4 to 5 experiments. In Fig. 20, no significant changes in bacterial density were observed in cultures treated with either GSH or ascorbic acid. This suggests that the effects seen in Figs. 17A and 19 are due to the NO release from the GSNO.

### 3F - Effect of GSNO:Asc (1:1) on killing of S. aureus biofilm grown on cell cultures established from sinonasal epithelial cells obtained from CRS patients

Sinonasal epithelial cell cultures were established from two patients (A and B) with CRS. The cell cultures were infected with *S. aureus* as described Example 3C. The basolateral medium was changed, the apical surface was washed with saline once and treated apically with 30 µl of saline, or saline containing 1.25 mM GSNO/Asc, and incubated for 4 hours. The apical surface was washed once with 0.2 ml of 0.15% bicarbonate in PBS and then with PBS. Cells were lysed in 1% Triton X-100 and serial dilutions of cell lysates were plated to determine the bacterial density. The results are shown in Fig. 21. The data represent the median and range from 4 or 6 experiments. In Fig. 21, * p<0.05, which is different from respective PBS treated cultures. The results in Fig. 21 show that the GSNO/Asc treatment significantly reduced the bacterial density on CRS cell cultures. More particularly, one application of the GSNO/Asc yielded an approximately 10-fold reduction in live bacteria counts on the surface of the cells.

### 3G - Effect of Ascorbic Acid on S. aureus biofilm growth on CF airway epithelial cells

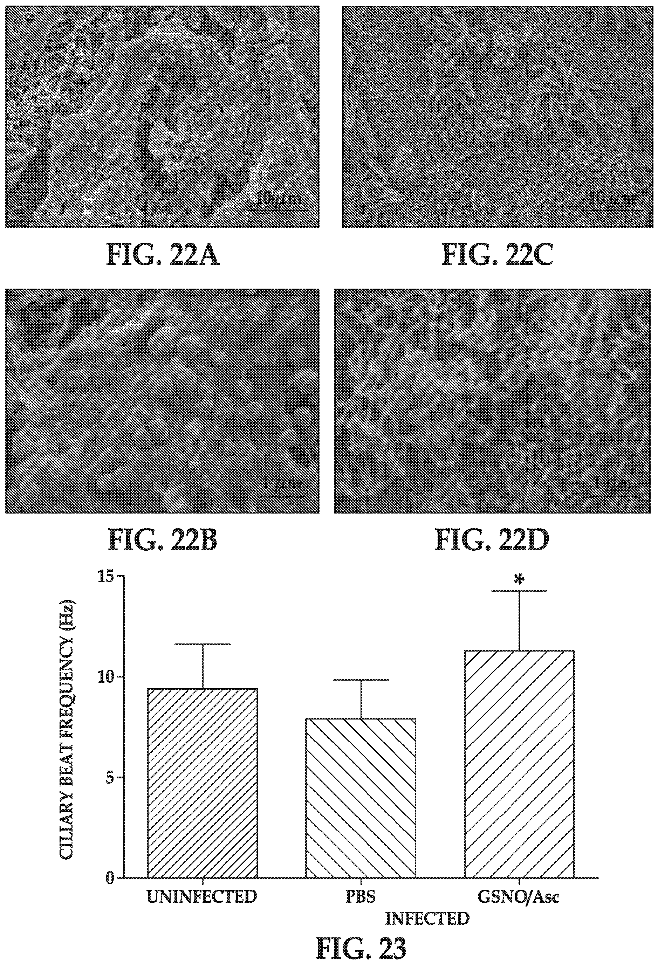

Apical surface of CF airway epithelial cells were infected apically with 10 µl of *S*. *aureus* (1 x 10¹⁰ CFU/ml) and incubated for 24 hours. The basolateral medium was changed, the apical surface was washed with saline and treated apically with 30 µl of PBS, or saline containing 1.25 mM GSNO/Asc and incubated for 3 hours. Cultures were washed once with PBS, fixed in glutaraldehyde and processed for scanning electron microscopy. Figs. 22A and 22B show bacterial biofilm on the surface of CF airway epithelial cells that were not treated with GSNO-Asc, while Figs. 22C and 22D show bacterial biofilm on the surface of CF airway epithelial cells that were treated with GSNO-Asc. In comparing Figs. 22A and 22B with Figs. 22C and 22D, there is comparatively less bacteria and the biofilm size is smaller for the sample treated with GSNO-Asc.

### 3H- Effect of Ascorbic Acid on Ciliary Beat Frequency

Control CF airway epithelial cells were uninfected. Other CF airway epithelial cells were infected apically with 10 µl of *S. aureus* (2.5 x 10³ CFU/ml) and incubated for 24 hours. The basolateral medium was changed, the apical surfaces were washed with saline, and the infected cultures were treated apically with 30 µl of PBS, or saline containing 1.25 mM GSNO/Asc, and incubated for 3 hours. The cultures were washed with 0.2 ml 0.15% sodium bicarbonate and imaged with high-speed video microscopy. Liquid from the apical surfaces was aspirated and 10 µl of media was added to prevent drying while imaging the cells with high speed video microscopy to quantify the ciliary beat frequency. In particular, ciliary beat frequency was measured in random fields using NIH image J software. The results are shown in Fig. 23. The data represent the median from 6 experiments, and * p<0.05, which is different from respective PBS treated and infected cultures. The results in Fig. 23 for the infected culture treated with PBS show that infection with *S. aureus* reduces ciliary beat frequency of the CF airway epithelial cells. The results in Fig. 23 for the infected culture treated with GSNO:Asc shows a tendency to improve ciliary beat frequency (when compared to the infected culture treated with PBS).

### EXAMPLE 4

Two catheter lock solutions were prepared in PBS (i.e., carrier and salt) with different GSNO and GSH concentrations. The first catheter lock solution included a 1:1 molar (equimolar) ratio of GSNO:GSH, with each of GSNO and GSH being at a concentration of 2 mM. The second catheter lock solution included a 1:1 molar (equimolar) ratio of GSNO:GSH, with each of GSNO and GSH being at a concentration of 10 mM.

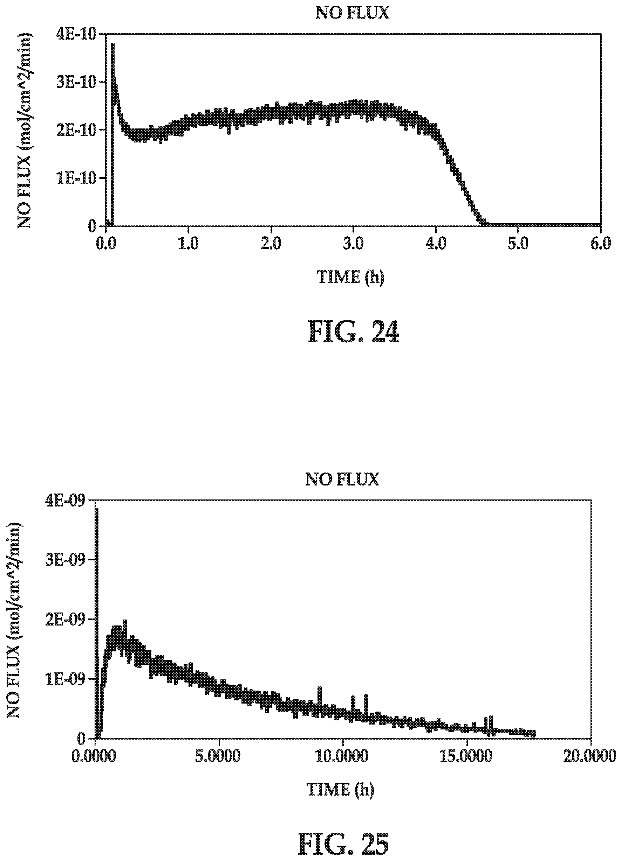

A silicone rubber catheter tubing (inner diameter = 1.58 mm, outer diameter = 3.18 mm, and volume 176 µL) was used and was closed at the distal end with a plug. The tubing was filled with the first catheter lock solution. NO permeated the silicone rubber catheter tubing, and thus the NO release from the outer surface of the tubing was monitored via chemiluminescence. The results are shown in Fig. 24. As depicted, the NO release was observed for at least 4 hours, and the flux was near the physiologically relevant level required for antiplatelet and antimicrobial activity.

A polyurethane (PU) catheter tubing was also used and was closed at the distal end with a plug. The PU tubing was filled with the second catheter lock solution. NO permeated the PU tubing, and thus the NO release from the outer surface of the tubing was monitored via chemiluminescence. The results are shown in Fig. 25. As depicted, the NO release was observed for at least 15 hours. The flux was somewhat above physiologically relevant levels initially (and thus there was high antimicrobial activity) and then the flux slowly decreased over time to the more physiologically relevant levels required for antiplatelet and antimicrobial activity.

## Claims

1. A nitric oxide (NO) generating formulation, comprising:
an S-nitrosothiol (RSNO) powder;
a salt; and
an additive to control or accelerate a rate of release of NO from RSNO after the RSNO powder, the salt, and the additive are dissolved into a liquid carrier,
wherein
a) the NO generating formulation is an antimicrobial sinonasal treatment formulation, and the salt is selected from the group consisting of sodium chloride, sodium bicarbonate, calcium chloride, a sodium phosphate buffer, a potassium phosphate buffer, and combinations thereof; or
b) the NO generating formulation is a catheter lock solution formulation, and the salt is selected from the group consisting of sodium chloride, sodium bicarbonate, a sodium phosphate buffer, a potassium phosphate buffer, and combinations thereof.

2. The NO generating formulation as defined in claim 1 a) wherein an amount of the RSNO powder in the antimicrobial sinonasal treatment formulation corresponds with a RSNO concentration ranging from about 25 µM to about 10 mM in an antimicrobial sinonasal treatment solution formed when the RSNO powder, the salt, and the additive are dissolved into a predetermined amount of the liquid carrier,
preferably the NO generating formulation further comprises the liquid carrier selected from the group consisting of water, a saline solution, a saline solution with sodium bicarbonate (NaHCO₃), a phosphate buffered saline solution (PBS), an aqueous solution with calcium chloride (CaCl₂), and an aqueous solution with a sodium phosphate buffer or with a potassium phosphate buffer,
or preferably the NO generating formulation further comprises a metal ion chelator selected from the group consisting of ethylenediaminetetraacetic acid (EDTA), ethylene glycol tetraacetic acid (EGTA), sodium citrate, and combinations thereof.

3. The NO generating formulation as defined in claim 1 b) wherein an amount of the RSNO powder in the catheter lock solution formulation corresponds with a RSNO concentration ranging from about 500 µM to about 50 mM in a catheter lock solution formed when the RSNO powder, the salt, and the additive are dissolved into a predetermined amount of the liquid carrier,
preferably the NO generating formulation further comprises an anticoagulant selected from the group consisting of heparin, ethylenediaminetetraacetic acid (EDTA), sodium citrate, and combinations thereof,
or preferably the generating formulation further comprises the liquid carrier selected from the group consisting of water, a saline solution, a saline solution with sodium bicarbonate (NaHCO₃), a phosphate buffered saline solution (PBS), and an aqueous solution with a sodium phosphate buffer or with a potassium phosphate buffer.

4. The NO generating formulation as defined in any one of claims 1 to 3 wherein a molar ratio of the RSNO to the additive ranges from 1:10 to 10:1.

5. The NO generating formulation as defined in any one of claims 1 to 4 wherein the additive is selected from the group consisting of reduced glutathione, cysteine, ascorbic acid or ascorbate, copper ions, zinc ions, zinc-oxide particles, an organoselenium species, and combinations thereof,
preferably wherein the organoselenium species is selected from the group consisting of selenocysteine and ebeselen.

6. The NO generating formulation as defined in any one of claims 1 to 5 wherein:
the RSNO powder, the salt, and the additive are part of a single powder composition; or
the RSNO powder, the salt, and the additive are part of a single pellet,
or wherein the RSNO powder is contained within a first container, and the salt and the additive are contained in a second container that is separate from the first container or the salt and the additive are contained, respectively, in a second container and a third container that are separate from the first container.

7. The NO generating formulation as defined in any one of claims 1 to 6 wherein the RSNO powder is selected from the group consisting of *S-*nitrosoglutathione (GSNO), S-nitroso-cysteine, S-nitroso-N-acetyl-penicillamine, *S-*nitroso-penicillamine, and S-nitroso-human serum albumin.

8. A nitric oxide (NO) generating kit, comprising;
a first powder composition including S-nitrosothiol (RSNO);
a second powder composition including an additive to control or accelerate a rate of release of the NO from the RSNO;
a salt present in the first powder composition or the second powder composition or a third powder composition; and
a container for dissolving the first and second powder compositions into a liquid carrier.

9. The NO generating kit as defined in claim 8 wherein the container is one of:
a nasal flush bottle; or
an ampule or a vial.

10. The NO generating kit as defined in claim 8 wherein one of:
the NO generating kit is an antimicrobial sinonasal treatment kit, and the salt is selected from the group consisting of sodium chloride, sodium bicarbonate, calcium chloride, a sodium phosphate buffer, a potassium phosphate buffer, and combinations thereof; or
the NO generating kit is a catheter lock solution kit, and the salt is selected from the group consisting of sodium chloride, sodium bicarbonate, a sodium phosphate buffer, a potassium phosphate buffer, and combinations thereof.

11. The NO generating kit as defined in claim 8 wherein one of:
the NO generating kit is an antimicrobial sinonasal treatment kit, and wherein:
the antimicrobial sinonasal treatment kit further comprises a metal ion chelator present in the first powder composition or the second powder composition; and
the metal ion chelator is selected from the group consisting of ethylenediaminetetraacetic acid (EDTA), ethylene glycol tetraacetic acid (EGTA), sodium citrate, and combinations thereof; or
the NO generating kit is a catheter lock solution kit, and wherein:
the catheter lock solution kit further comprises an anticoagulant selected from the group consisting of heparin, ethylenediaminetetraacetic acid (EDTA), sodium citrate, and combinations thereof.

12. The NO generating kit as defined in claim 8 wherein one of:
the NO generating kit is an antimicrobial sinonasal treatment kit, and an amount of the RSNO in the first powder composition corresponds with a RSNO concentration ranging from about 25 µM to about 10 mM in an antimicrobial sinonasal treatment solution formed when the first and second powder compositions are dissolved into a predetermined amount of the liquid carrier; or
the NO generating kit is a catheter lock solution kit, and an amount of the RSNO in the first powder composition corresponds with a RSNO concentration ranging from about 500 µM to about 50 mM in a catheter lock solution formed when the first and second powder compositions are dissolved into a predetermined amount of the liquid carrier.

13. The NO generating kit as defined in any one of claims 8 to 12 wherein:
the RSNO is selected from the group consisting of *S*-nitrosoglutathione (GSNO), *S*-nitroso-cysteine, *S*-nitroso-N-acetyl-penicillamine, *S*-nitroso-penicillamine, and *S*-nitroso-human serum albumin; and
the additive is selected from the group consisting of reduced glutathione, cysteine, ascorbic acid or ascorbate, copper ions, zinc ions, zinc-oxide particles, an organoselenium species, and combinations thereof.

14. The NO generating kit as defined in claim 8, further comprising water as the liquid carrier,
preferably wherein the water is mixed with the salt to form a sodium bicarbonate/saline solution including 0.16 M sodium chloride (NaCl) and 0.065 M sodium bicarbonate (NaHCO₃) in the water.

15. The NO generating kit as defined in claim 8 wherein:
the salt is contained in the third powder composition; and
the first, second and third powder compositions are each contained in separate containers, or
the salt is present in the first powder composition or the second powder composition; and
the first powder composition and the second powder composition are combined in a single container, or in a single pellet, or in a single tablet.

## Patentansprüche

1. Stickstoffoxid(nitric oxide - NO)-erzeugende Formulierung, die Folgendes umfasst:
ein S-Nitrosothiol(RSNO)-Pulver;
ein Salz; und
ein Additiv, um eine Freisetzungsrate von NO aus RSNO zu steuern oder beschleunigen, nachdem das RSNO-Pulver, das Salz und das Additiv in einer flüssigen Trägersubstanz gelöst worden sind,
wobei
a) die NO-erzeugende Formulierung eine Formulierung einer antimikrobiellen sinunasalen Behandlung ist und das Salz aus der Gruppe ausgewählt ist, die aus Natriumchlorid, Natriumbicarbonat, Calciumchlorid, einem Natriumphosphatpuffer, einem Kaliumphosphatpuffer und Kombinationen davon besteht; oder
b) die NO-erzeugende Formulierung eine
Katheterverschlusslösungsformulierung ist und das Salz aus der Gruppe ausgewählt ist, die aus Natriumchlorid, Natriumbicarbonat, einem Natriumphosphatpuffer, einem Kaliumphosphatpuffer und Kombinationen davon besteht.

2. NO-erzeugende Formulierung nach Anspruch 1 a), wobei eine Menge des RSNO-Pulvers in der Formulierung der antimikrobiellen sinunasalen Behandlung einer RSNO-Konzentration in einem Bereich von etwa 25 µM bis etwa 10 mM in einer Lösung der antimikrobiellen sinunasalen Behandlung entspricht, die ausgebildet wird, wenn das RSNO-Pulver, das Salz und das Additiv in einer zuvor bestimmten Menge der flüssigen Trägersubstanz gelöst werden,
vorzugsweise die NO-erzeugende Formulierung ferner die flüssige Trägersubstanz umfasst, die aus der Gruppe ausgewählt ist, die aus Wasser, einer Kochsalzlösung, einer Kochsalzlösung mit Natriumbicarbonat (NaHCO₃), einer phosphatgepufferten physiologischen Kochsalzlösung (phosphate buffered saline - PBS), einer wässrigen Lösung mit Calciumchlorid (CaCl₂) und einer wässrigen Lösung mit einem Natriumphosphatpuffer oder mit einem Kaliumphosphatpuffer besteht,
oder vorzugsweise die NO-erzeugende Formulierung ferner einen Metallionenchelatbildner umfasst, der aus der Gruppe ausgewählt ist, die aus Ethylendiamintetraessigsäure (ethylenediaminetetraacetic acid - EDTA), Ethylenglycoltetraessigsäure (ethylene glycol tetraacetic acid - EGTA), Natriumcitrat und Kombinationen davon besteht.

3. NO-erzeugende Formulierung nach Anspruch 1 b), wobei eine Menge des RSNO-Pulvers in der Katheterverschlusslösungsformulierung einer RSNO-Konzentration in einem Bereich von etwa 500 µM bis etwa 50 mM in einer Katheterverschlusslösung entspricht, die ausgebildet wird, wenn das RSNO-Pulver, das Salz und das Additiv in einer zuvor bestimmten Menge der flüssigen Trägersubstanz gelöst werden,
vorzugsweise die NO-erzeugende Formulierung ferner ein Antikoagulans umfasst, das aus der Gruppe ausgewählt ist, die aus Heparin, Ethylendiamintetraessigsäure (EDTA), Natriumcitrat und Kombinationen davon besteht,
oder vorzugsweise die erzeugende Formulierung ferner die flüssige Trägersubstanz umfasst, die aus der Gruppe ausgewählt ist, die aus Wasser, einer Kochsalzlösung, einer Kochsalzlösung mit Natriumbicarbonat (NaHCO₃), einer phosphatgepufferten physiologischen Kochsalzlösung (PBS) und einer wässrigen Lösung mit einem Natriumphosphatpuffer oder mit einem Kaliumphosphatpuffer besteht.

4. NO-erzeugende Formulierung nach einem der Ansprüche 1 bis 3, wobei ein Molverhältnis des RSNO zu dem Additiv in einem Bereich von 1 : 10 bis 10 : 1 liegt.

5. NO-erzeugende Formulierung nach einem der Ansprüche 1 bis 4, wobei das Additiv aus der Gruppe ausgewählt ist, die aus reduziertem Glutathion, Cystein, Ascorbinsäure oder Ascorbat, Kupferionen, Zinkionen, Zinkoxidpartikeln, einer Organoselenspezies und Kombinationen davon besteht,
vorzugsweise wobei die Organoselenspezies aus der Gruppe ausgewählt ist, die aus Selenocystein und Ebeselen besteht.

6. NO-erzeugende Formulierung nach einem der Ansprüche 1 bis 5, wobei:
das RSNO-Pulver, das Salz und das Additiv Teil einer einzelnen Pulverzusammensetzung sind; oder
das RSNO-Pulver, das Salz und das Additiv Teil eines einzelnen Pellets sind,
oder wobei das RSNO-Pulver innerhalb eines ersten Behälters enthalten ist und das Salz und das Additiv in einem zweiten Behälter enthalten sind, der von dem ersten Behälter getrennt ist, oder das Salz und das Additiv jeweils in einem zweiten Behälter und einem dritten Behälter enthalten sind, die von dem ersten Behälter getrennt sind.

7. NO-erzeugende Formulierung nach einem der Ansprüche 1 bis 6, wobei das RSNO-Pulver aus der Gruppe ausgewählt ist, die aus S-Nitrosoglutathion (GSNO), S-Nitroso-Cystein, S-Nitroso-N-Acetyl-Penicillamin, S- Nitroso-Penicillamin und S-Nitroso-Humanserumalbumin besteht.

8. Stickstoffoxid(NO)-erzeugendes Kit, das Folgendes umfasst;
eine erste Pulverzusammensetzung, die S-Nitrosothiol (RSNO) beinhaltet;
eine zweite Pulverzusammensetzung, die ein Additiv beinhaltet, um eine Freisetzungsrate des NO aus dem RSNO zu steuern oder beschleunigen;
ein Salz, das in der ersten Pulverzusammensetzung oder der zweiten Pulverzusammensetzung oder einer dritten Pulverzusammensetzung vorhanden ist; und
einen Behälter zum Auflösen der ersten und der zweiten Pulverzusammensetzung in einer flüssigen Trägersubstanz.

9. NO-erzeugendes Kit nach Anspruch 8, wobei der Behälter Folgendes ist:
eine Nasenspülflasche; oder
eine Ampulle oder ein Vial.

10. NO-erzeugendes Kit nach Anspruch 8, wobei:
das NO-erzeugende Kit ein Kit der antimikrobiellen sinunasalen Behandlung ist und das Salz aus der Gruppe ausgewählt ist, die aus Natriumchlorid, Natriumbicarbonat, Calciumchlorid, einem Natriumphosphatpuffer, einem Kaliumphosphatpuffer und Kombinationen davon besteht; oder
das NO-erzeugende Kit ein Katheterverschlusslösungskit ist und das Salz aus der Gruppe ausgewählt ist, die aus Natriumchlorid, Natriumbicarbonat, einem Natriumphosphatpuffer, einem Kaliumphosphatpuffer und Kombinationen davon besteht.

11. NO-erzeugendes Kit nach Anspruch 8, wobei:
das NO-erzeugende Kit ein Kit der antimikrobiellen sinunasalen Behandlung ist und wobei:
das Kit der antimikrobiellen sinunasalen Behandlung ferner einen Metallionenchelatbildner umfasst, der in der ersten Pulverzusammensetzung oder der zweiten Pulverzusammensetzung vorhanden ist; und
der Metallionenchelatbildner aus der Gruppe ausgewählt ist, die aus Ethylendiamintetraessigsäure (EDTA), Ethylenglycoltetraessigsäure (EGTA), Natriumcitrat und Kombinationen davon besteht; oder
das NO-erzeugende Kit ein Katheterverschlusslösungskit ist, und wobei:
das Katheterverschlusslösungskit ferner ein Antikoagulans umfasst, das aus der Gruppe ausgewählt ist, die aus Heparin, Ethylendiamintetraessigsäure (EDTA), Natriumcitrat und Kombinationen davon besteht.

12. NO-erzeugendes Kit nach Anspruch 8, wobei:
das NO-erzeugende Kit ein Kit der antimikrobiellen sinunasalen Behandlung ist und eine Menge des RSNO in der ersten Pulverzusammensetzung einer RSNO-Konzentration in einem Bereich von etwa 25 µM bis etwa 10 mM in einer Lösung der antimikrobiellen sinunasalen Behandlung entspricht, die ausgebildet wird, wenn die erste und die zweite Pulverzusammensetzung in einer zuvor bestimmten Menge der flüssigen Trägersubstanz gelöst werden; oder
das NO-erzeugende Kit ein Katheterverschlusslösungskit ist und eine Menge des RSNO in der ersten Pulverzusammensetzung einer RSNO-Konzentration in einem Bereich von etwa 500 µM bis etwa 50 mM in einer Katheterverschlusslösung entspricht, die ausgebildet wird, wenn die erste und die zweite Pulverzusammensetzung in einer zuvor bestimmten Menge der flüssigen Trägersubstanz gelöst werden.

13. NO-erzeugendes Kit nach einem der Ansprüche 8 bis 12, wobei:
das RSNO aus der Gruppe ausgewählt ist, die aus S-Nitrosoglutathion (GSNO), S-Nitroso-Cystein, S-Nitroso-N-Acetyl-Penicillamin, S-Nitroso-Penicillamin und S-Nitroso-Humanserumalbumin besteht; und
das Additiv aus der Gruppe ausgewählt ist, die aus reduziertem Glutathion, Cystein, Ascorbinsäure oder Ascorbat, Kupferionen, Zinkionen, Zinkoxidpartikeln, einer Organoseleniumspezies und Kombinationen davon besteht.

14. NO-erzeugendes Kit nach Anspruch 8, das ferner Wasser als die flüssige Trägersubstanz umfasst,
vorzugsweise wobei das Wasser mit dem Salz vermischt ist, um eine Natriumbicarbonat/Kochsalzlösung auszubilden, die 0,16 M Natriumchlorid (NaCl) und 0,065 M Natriumbicarbonat (NaHCO₃) in dem Wasser beinhaltet.

15. NO-erzeugendes Kit nach Anspruch 8, wobei:
das Salz in der dritten Pulverzusammensetzung enthalten ist; und
die erste, die zweite und die dritte Pulverzusammensetzung jeweils in getrennten Behältern enthalten sind oder
das Salz in der ersten Pulverzusammensetzung oder der zweiten Pulverzusammensetzung vorhanden ist; und
die erste Pulverzusammensetzung und die zweite Pulverzusammensetzung in einem einzelnen Behälter oder in einem einzelnen Pellet oder in einer einzelnen Tablette kombiniert sind.

## Revendications

1. Formulation générant de l'oxyde nitrique (NO), comprenant :
une poudre de S-nitrosothiol (RSNO) ;
un sel ; et
un additif pour réguler ou accélérer une vitesse de libération du NO à partir du RSNO après dissolution de la poudre de RSNO, le sel et l'additif dans un véhicule liquide,
dans laquelle
a) la formulation générant du NO est une formulation antimicrobienne de traitement naso-sinusien, et le sel est choisi dans le groupe constitué de chlorure de sodium, de bicarbonate de sodium, de chlorure de calcium, d'un tampon de phosphate de sodium, d'un tampon de phosphate de potassium, et de leurs combinaisons ; ou
b) la formulation générant du NO est une formulation de solution de verrou de cathéter, et le sel est choisi dans le groupe constitué de chlorure de sodium, de bicarbonate de sodium, d'un tampon de phosphate de sodium, d'un tampon de phosphate de potassium et de leurs combinaisons.

2. Formulation générant du NO selon la revendication 1 a) dans laquelle une quantité de poudre de RSNO dans la formulation de traitement antimicrobien naso-sinusien correspond à une concentration de RSNO allant d'environ 25 µM à environ 10 mM dans une solution de traitement antimicrobien naso-sinusien formée lorsque la poudre de RSNO, le sel et l'additif sont dissous dans une quantité prédéterminée du véhicule liquide,
de préférence, la formulation générant du NO comprend en outre le véhicule liquide choisi dans le groupe constitué d'eau, d'une solution saline, d'une solution saline avec du bicarbonate de sodium (NaHCO₃), d'une solution saline tamponnée au phosphate (PBS), d'une solution aqueuse avec du chlorure de calcium (CaCl₂), et d'une solution aqueuse avec un tampon de phosphate de sodium ou avec un tampon de phosphate de potassium,
ou de préférence, la formulation générant du NO comprend en outre un chélateur d'ions métalliques choisi dans le groupe constitué d'acide éthylènediaminetétraacétique (EDTA), d'acide éthylèneglycol tétraacétique (EGTA), de citrate de sodium et de leurs combinaisons.

3. Formulation générant du NO selon la revendication 1 b), dans laquelle une quantité de poudre de RSNO dans la formulation de solution de verrou de cathéter correspond à une concentration de RSNO allant d'environ 500 µM à environ 50 mM dans une solution de verrou de cathéter formée lorsque la poudre de RSNO, le sel et l'additif sont dissous dans une quantité prédéterminée du véhicule liquide,
de préférence, la formulation générant du NO comprend en outre un anticoagulant choisi dans le groupe constitué d'héparine, d'acide éthylènediaminetétraacétique (EDTA), de citrate de sodium et de leurs combinaisons,
ou de préférence, la formulation génératrice comprend en outre le véhicule liquide choisi dans le groupe constitué d'eau, d'une solution saline, d'une solution saline avec du bicarbonate de sodium (NaHCO₃), d'une solution saline tamponnée au phosphate (PBS) et d'une solution aqueuse avec un tampon de phosphate de sodium ou avec un tampon de phosphate de potassium.

4. Formulation générant du NO selon l'une quelconque des revendications 1 à 3, dans laquelle un rapport molaire du RSNO à l'additif se trouve dans une plage allant de 1:10 à 10:1.

5. Formulation générant du NO selon l'une quelconque des revendications 1 à 4, dans laquelle l'additif est choisi dans le groupe constitué de glutathion réduit, de cystéine, d'acide ascorbique ou d'ascorbate, d'ions de cuivre, d'ions de zinc, de particules d'oxyde de zinc, d'une espèce d'organosélénium, et de leurs combinaisons,
de préférence dans lequel l'espèce d'organosélénium est choisie dans le groupe constitué de sélénocystéine et d'ebselen.

6. Formulation générant du NO selon l'une quelconque des revendications 1 à 5, dans laquelle :
la poudre de RSNO, le sel et l'additif font partie d'une même composition de poudre ; ou
la poudre de RSNO, le sel et l'additif font partie d'une même pastille,
ou dans lequel la poudre de RSNO est contenue dans un premier conteneur, et le sel et l'additif sont contenus dans un deuxième conteneur qui est séparé du premier conteneur ou le sel et l'additif sont contenus, respectivement, dans un deuxième conteneur et un troisième conteneur qui sont séparés du premier conteneur.

7. Formulation générant du NO selon l'une quelconque des revendications 1 à 6, dans laquelle la poudre de RSNO est choisie dans le groupe constitué de S-nitrosoglutathion (GSNO), de S-nitroso-cystéine, de S-nitroso-N-acétyl-pénicillamine, de S-nitrosopénicillamine et de S-nitroso-albumine sérique humaine.

8. Kit de génération d'oxyde nitrique (NO), comprenant ;
une première composition de poudre comportant du S-nitrosothiol (RSNO) ;
une deuxième composition de poudre comportant un additif pour réguler ou accélérer une vitesse de libération du NO à partir du RSNO ;
un sel présent dans la première composition de poudre ou la deuxième composition de poudre ou une troisième composition de poudre ; et
un récipient pour dissoudre les première et deuxième compositions de poudre dans un véhicule liquide.

9. Kit de génération de NO selon la revendication 8, dans lequel le conteneur est l'un parmi :
une bouteille de rinçage nasale ; ou
une ampoule ou un flacon.

10. Kit de génération de NO selon la revendication 8, dans lequel l'un parmi :
le kit de génération de NO est un kit de traitement naso-sinusien antimicrobien, et le sel est choisi dans le groupe constitué de chlorure de sodium, de bicarbonate de sodium, de chlorure de calcium, d'un tampon de phosphate de sodium, d'un tampon de phosphate de potassium et de leurs combinaisons ; ou
le kit de génération de NO est un kit de solution de verrou de cathéter, et le sel est choisi dans le groupe constitué de chlorure de sodium, de bicarbonate de sodium, d'un tampon de phosphate de sodium, d'un tampon de phosphate de potassium et de leurs combinaisons.

11. Kit de génération de NO tel que défini dans la revendication 8, dans lequel l'un parmi :
le kit de génération de NO est un kit de traitement antimicrobien naso-sinusien, et dans lequel :
le kit de traitement antimicrobien naso-sinusien comprend en outre un chélateur d'ions métalliques présent dans la première composition de poudre ou la deuxième composition de poudre ; et
le chélateur d'ions métalliques est choisi dans le groupe constitué d'acide éthylènediaminetétraacétique (EDTA), d'acide éthylèneglycol tétraacétique (EGTA), de citrate de sodium et de leurs combinaisons ; ou
le kit de génération de NO est un kit de solution de verrou de cathéter, et dans lequel :
le kit de solution de verrou de cathéter comprend en outre un anticoagulant choisi dans le groupe constitué d'héparine, d'acide éthylènediaminetétraacétique (EDTA), de citrate de sodium et de leurs combinaisons.

12. Kit de génération de NO selon la revendication 8, dans lequel l'un parmi :
le kit de génération de NO est un kit de traitement antimicrobien naso-sinusien, et une quantité de RSNO dans la première composition de poudre correspond à une concentration de RSNO allant d'environ 25 µM à environ 10 mM dans une solution de traitement antimicrobien naso-sinusien formée lorsque les première et deuxième compositions de poudre sont dissous dans une quantité prédéterminée du véhicule liquide ; ou
le kit de génération de NO est un kit de solution de verrou de cathéter, et une quantité de RSNO dans la première composition de poudre correspond à une concentration de RSNO allant d'environ 500 µM à environ 50 mM dans une solution de verrou de cathéter formée lorsque les première et deuxième compositions de poudre sont dissous dans une quantité prédéterminée du véhicule liquide.

13. Kit de génération de NO selon l'une quelconque des revendications 8 à 12, dans lequel :
le RSNO est choisi dans le groupe constitué de *S*-nitrosoglutathione (GSNO), de S-nitroso-cystéine, de S-nitroso-N-acétyl-pénicillamine, de S-nitrosopénicillamine et de S-nitroso-albumine sérique humaine ; et
l'additif est choisi dans le groupe constitué de glutathion réduit, la cystéine, d'acide ascorbique ou d'ascorbate, d'ions de cuivre, d'ions de zinc, de particules d'oxyde de zinc, d'une espèce d'organosélénium et de leurs combinaisons.

14. Kit de génération de NO selon la revendication 8, comprenant en outre de l'eau comme véhicule liquide,
de préférence dans lequel l'eau est mélangée avec le sel pour former une solution de bicarbonate de sodium/solution saline comportant 0,16 M de chlorure de sodium (NaCl) et 0,065 M de bicarbonate de sodium (NaHCO₃) dans l'eau.

15. Kit de génération de NO selon la revendication 8, dans lequel :
le sel est contenu dans la troisième composition de poudre ; et
les première, deuxième et troisième compositions de poudre sont chacune contenues dans des récipients séparés, ou
le sel est présent dans la première composition de poudre ou la deuxième composition de poudre ; et
la première composition de poudre et la deuxième composition de poudre sont réunies dans un même récipient, ou dans une seule pastille, ou dans un seul comprimé.
